# EUROPÄISCHE PATENTSCHRIFT

(11) **EP 3 293 171 B1**
(45) Veröffentlichungstag und Bekanntmachung des Hinweises auf die Patenterteilung: **28.08.2019**
(21) Anmeldenummer: 17189153.4
(22) Anmeldetag: 04.09.2017
(51) Int. Cl.: C07C 45/50, C07C 2/24, C07C 5/32, C07C 5/48

(54) **VERFAHREN ZUR FLEXIBLEN HERSTELLUNG VON ALDEHYDEN**
PROCESS FOR THE FLEXIBLE PREPARATION OF ALDEHYDES
PROCÉDÉ POUR LA PRODUCTION FLEXIBLE D'ALDÉHYDES

(30) Priorität: 12.09.2016 EP 16188267
(43) Veröffentlichungstag der Anmeldung: 14.03.2018
(73) Patentinhaber: Evonik Degussa GmbH, 45128 Essen (DE)
(72) Erfinder: Stochniol, Guido, 45721 Haltern am See (DE); Wolff, Andreas, 45657 Recklinghausen (DE); Peitz, Stephan, 45739 Oer-Erkenschwick (DE)

(56) Entgegenhaltungen:
- EP-A1- 0 820 973
- DE-A1- 10 251 262
- US-A1- 2011 282 092

## Beschreibung

Die Erfindung befasst sich mit der Frage wie alternative Rohstoffe bei der Herstellung C₄-basierter Aldehyde erschlossen werden können.

Kohlenwasserstoffe sind chemische Verbindungen, die ausschließlich aus Kohlenstoff und Wasserstoff bestehen. Alkene (synonym: Olefine) sind Kohlenwasserstoffe, die eine C=C Doppelbindung im Molekül aufweisen. Alkane (synonym: Paraffine) sind dagegen Kohlenwasserstoffe, die nur Einfachbindungen aufweisen. Sie werden deswegen auch als gesättigt bezeichnet. Aufgrund der unterschiedlichen Bindungstypen sind die Alkene deutlich reaktiver als die Alkane. Deswegen sind Alkene chemisch besser nutzbar und entsprechend wertvoller als Alkane.

In der organischen Chemie werden Kohlenwasserstoffe häufig nach der Anzahl ihrer Kohlenstoffatome pro Molekül bezeichnet, indem der jeweiligen Substanzklasse das Präfix Cₙ vorangestellt wird. Dabei bezeichnet n die jeweilige Anzahl der Kohlenstoffatome in einem Molekül. So sind C₄-Olefine zu verstehen als Substanzen der Klasse der Alkene mit vier Kohlenstoffatomen. C₈-Olefine weisen dementsprechend acht Kohlenstoffatome pro Molekül auf. Soweit im Folgenden das Präfix Cₙ₊ verwendet wird, ist von einer Substanzklasse die Rede, die mehr als n Kohlenstoffatome pro Molekül aufweist. Ein C₄₊-Olefin hat demnach mindestens fünf Kohlenstoffatome.

Aufgrund unterschiedlicher Anordnungs- und Verknüpfungsmöglichkeiten der Kohlenstoff- und Wasserstoffatome existieren innerhalb den hier besprochenen Substanzklassen mehrere Isomere, welche dieselbe Anzahl an Kohlenstoffatomen aufweisen. So existieren beispielsweise zwei Alkane mit jeweils vier Kohlenstoffatomen, nämlich n-Butan und Isobutan. Da bei den Alkenen die Kombinationsvielfalt größer ist, sind noch mehr Isomere möglich. So kommen insgesamt vier Olefine mit vier Kohlenstoffatomen vor, nämlich Isobuten, 1-Buten, cis-2-Buten und trans-2-Buten. Die drei linearen Butene 1-Buten, cis-2-Buten und trans-2-Buten werden oft zusammenfassend als n-Buten bezeichnet. Bei den C₃-Kohlenwasserstoffen gibt es hingegen jeweils nur ein Isomer, nämlich das Alkan mit drei Kohlenstoffatomen Propan und das C₃-Alken Propen. Bei den längerkettigen Kohlenwasserstoffen C₅₊ nimmt die Vielfalt der Isomere stark zu. Trotz der identischen Anzahl an Kohlenstoffatomen weisen Isomere unterschiedliche Stoffeigenschaften auf, die für ihre industrielle Nutzung relevant sind.

Die Substanzklasse der Aldehyde umfasst Stoffe, die aufgrund ihrer hohen Reaktivität als Ausgangssubstanz für die Herstellung unterschiedlicher Spezialchemikalien genutzt werden wie etwa Schmiermittel, Weichmacher und Detergenzien. Aldehyde können auch als Riechstoffe eingesetzt werden.

Aldehyde werden aus Alkenen und Synthesegas, das ist eine Mischung aus Wasserstoff und Kohlenmonoxid, hergestellt. Dieser Vorgang heißt Hydroformylierung oder Oxo-Reaktion. Dabei wird die Anzahl an Kohlenstoffatomen um eins erhöht. Aus einem C₄-Olefin entsteht auf diese Weise durch Hydroformylierung ein C₅-Aldehyd (Pentanal).

Aldehyde mit höherer Kohlenstoffzahl können dadurch erzeugt werden, dass man entweder niedrigere Aldehyde miteinander zu höheren Aldehyden umsetzt (Aldolkondensation) oder erst Olefine mit sich selbst umsetzt (Oligomerisierung) und die dabei erhaltenen Olefin-Oligomere sodann hydroformyliert.

So kann ein C₁₀-Aldehyd dadurch erhalten werden, dass ein C₄-Olefin zum C₅-Aldehyd hydroformyliert und dieses sodann durch Aldolkondensation mit sich selbst zum C₁₀-Aldehyd (Decanal) umgesetzt wird. Aus C₄-Olefin kann auch C₉-Aldehyd hergestellt werden, wenn man erst durch Oligomerisierung zu einem C₈-Olefin umsetzt und dieses anschließend zum C₉-Aldehyd hydroformyliert.

Aus C₄-Olefinen lassen sich somit sowohl Aldehyde mit fünf als auch mit neun Kohlenstoffatomen herstellen; mit anschließender Aldolkondensation der Pentanale auch C₁₀-Aldehyde. Dies wird in der industriellen Praxis in komplex verschalteten Verbundanlagen auch so betrieben:
DE102008007081A1 beschreibt ein Verfahren zur Verwertung von C₄-Gemischen, die mindestens 1-Buten, Isobuten, Butane, 2-Butene und mehrfach ungesättigte C₄-Kohlenwasserstoffe enthalten. Beiläufig wird erwähnt, dass sich aus dem separiertem n-Buten über Oligomerisierung und Hydroformylierung C₉-, C₁₃- und C₁₇-Aldehyde herstellen lassen, währenddessen das ebenfalls gewonnene hochreine 1-Buten sich unter anderem für die Herstellung von Valeraldehyd eignet.

Dieses Verfahren nutzt als Rohstoffquelle so genannte C4-Schnitte, die als "Crack-C4" aus Steamcrackern oder als "FCC-C4" aus fluid-katalytischen Crackern stammen. Solche Cracker werden im Wesentlichen mit Naphtha bzw. VGO (Vacuum Gas Oil) beschickt, welche wiederum aus der Destillation von Rohöl stammen. Da Crack-C4 und FCC-C4 in der Wertschöpfungskette der Petrochemie Produkte von Crackprozessen sind, sind die Preise für diese Rohstoffe aufgrund ihrer Abhängigkeit vom Erdölpreis entsprechend volatil. Darüber hinaus geht die Verfügbarkeit von hochwertigen Crack-C4 ständig zurück, da die Fahrweise der Streamcracker zu Ungunsten der C₄-Ausbeute auf die Produktion der C₂- und C₃-Olefine Ethen und Propen hin optimiert wird. Ein Nachteil des in DE102008007081A1 beschriebenen Verfahrens ist mithin in seiner Abhängigkeit von einer speziellen Rohstoffbasis zu sehen.

Ein weiterer Nachteil dieses Verfahrens ist, dass das in verwendeten C₄-Gemischen mitunter signifikant enthaltene n-Butan und Isobutan sich in dem Prozess inert verhält und deswegen nicht stofflich genutzt wird. Im Interesse der CO₂-Bilanz des Verfahrens ist es, möglichst alle im Einsatzstoffgemisch enthalten C-Atome nachhaltig chemisch zu verwerten und sie tunlichst nicht zu verbrennen. Die Ressourceneffizienz des aus DE102008007081A1 bekannten Verfahrens ist somit verbesserungsfähig.

Eine andere Rohstoffbasis nutzt das in EP0820974B1 beschriebene Verfahren. Verarbeitet werden dort so genannte "Feldbutane", das sind C₄-Fraktionen der "feuchten" Anteile des Erdgases und der Erdölbegleitgase, die durch Trocknung und Abkühlung auf etwa -30°C in flüssiger Form aus den Gasen abgetrennt werden. Durch Tieftemperaturdestillation gewinnt man daraus die Feldbutane, deren Zusammensetzung je nach Lagerstätte schwankt, die jedoch im Allgemeinen etwa 30 % Isobutan, und etwa 65 % n-Butan enthalten. Weitere Bestandteile sind in der Regel etwa 2 % Kohlenwasserstoffe mit weniger als vier C-Atomen und etwa 3 % C₄₊- Kohlenwasserstoffe.

Die Feldbutane werden dehydriert, sodass ein Gemisch entsteht, welches unter anderem n-Buten und Isobuten enthält. Dieses Gemisch wird aufgearbeitet und das dabei separierte n-Buten durch Oligomerisierung im Wesentlichen in C₈-Olefine und daneben auch in C₁₂ Olefine umgesetzt. Die C₈- und C₁₂ -Olefine werden durch Hydroformylierung und Hydrierung in C₉ - und C₁₃ - Alkohole überführt, sodass vor der Hydrierung entsprechende Aldehyde vorliegen müssen. C₅- Aldehyde werden indes nicht hergestellt. Nachteil dieses Verfahrens ist, dass es auf einen kontinuierlichen Absatz von C₉ und C₁₃ Alkohole angewiesen ist, um die bezogenen Feldbutane verwerten zu können. Da Feldbutane anderweitig kaum gehandelt werden, ist der Bezug von Feldbutanen nur über langfristige, kontinuierliche Lieferverträge möglich. Auch hier besteht also eine Abhängigkeit von einem Spezialrohstoff, die jedoch durch eine Abnehmerabhängigkeit verstärkt wird.

In Hinblick auf EP0820974B1 besteht also das Bedürfnis, durch technische Maßnahmen sowohl eine größere Freiheit in Bezug auf die Wahl der Rohstofflieferanten zu gewinnen als auch auf schwankende Nachfrage der Abnehmer reagieren zu können.

Wiederum eine andere Rohstoffquelle zur Aldehydherstellung wird in US2006/0122436A1 erschlossen. Die in dieser Veröffentlichung genutzten Alkane stammen aus LPG.

LPG (Liquefied Petroleum Gas) ist eine international gängige Handelsbezeichnung für ein flüssiges Gemisch aus C₃- und/oder C₄-Kohlenwasserstoffen, welches als Nebenprodukt bei der Gewinnung von Erdöl oder Erdgas an dessen Lagerstätte oder bei der Aufarbeitung von Rohöl in der Raffinerie anfällt. Die genaue Zusammensetzung von LPG hängt stark von seiner Herkunft ab; seine wesentlichen Bestandteile sind meist Propan und Butane.

Es existiert ein globales, auf LPG basierendes Ökosystem, welches dieses Produkt fördert, transportiert und hauptsächlich als Treib- und Brennstoff vermarktet.

Eine umfassende Einführung in die LPG Technologie und darauf aufbauende Wirtschaft bieten:
Thompson, S. M., Robertson, G. and Johnson, E.: Liquefied Petroleum Gas. Ullmann's Encyclopedia of Industrial Chemistry. Published Online: 15 JUL 2011. DOI: 10.1002/14356007.a15_347.pub2

US2006/0122436A1 offenbart nun zwei Wege, wie aus LPG Aldehyde bzw. Alkohole hergestellt werden können; vgl. dort die Ansprüche 1 und 7.

Bei dem ersten Verfahren nach Anspruch 1 werden Cₙ-Aldehyde aus Cₙ₋₁ Alkanen hergestellt. n steht dabei für eine ganze Zahl von 4 bis 20. Dementsprechend werden im Falle von n=5 C₅-Aldehyde aus C₄-Alkanen und im Falle von n=9 C₉-Aldehyde aus C₈-Alkanen hergestellt. Dies geschieht beispielsweise bei C₄-Alkanen dadurch, dass das im LPG enthaltende Butan zunächst zu Buten und Nebenbestandteile dehydriert wird. Nach Abtrennung der Nebenbestanteile werden die Butene zu Pentanalen hydroformyliert. Die Pentanale werden durch Aldolkondensation in Decanale überführt.

Bei dem zweiten Verfahren werden aus Cₙ₋₁ Alkanen über Cₙ-Aldehyde, C₂ₙ-Aldehyde und C₂ₙ₋₁-Aldehyde die entsprechenden C₂ₙ- und C₂ₙ₋₁-Alkohole hergestellt. Im Falle von n=5 werden dementsprechend aus C₄-Alkanen C₅-, C₉- und C₁₀-Alkohole hergestellt. Dies geschieht grundsätzlich genauso wie im ersten Prozess jedoch mit dem Unterschied, dass die Hydroformylierung beispielsweise der C₄-Alkene zu den Pentanalen explizit nur unter Teilumsatz erfolgt; vgl. Schritt 7c. Die Pentanale werden von den nicht umgesetzten Butenen abgetrennt (Schritt 7d) und zu Decanalen aldolkondensiert, um anschließend mittels katalystischer Hydrierung Decanole herzustellen (Schritte 7e und 7f). Die nicht umgesetzten Butene werden einer Oligomerisierung (Schritt 7g) unterworfen, sodass C₈-Olefine erhalten werden. Diese werde dann zu C₉-Aldehyden hydroformyliert (Schritt 7h) und anschließend zu C₉-Alkoholen hydriert (Schritt 7i).

Ein konzeptioneller Nachteil dieses Verfahrens ist, dass die Oligomerisierung (von Cₙ₋₁ zu C₂ₙ₋₂) und die anschließende zweite Hydroformylierung der Oligomere (von C₂ₙ₋₂ zu C₂ₙ₋₁) stromabwärts hinter der ersten Hydroformylierung (von Cₙ₋₁ zu Cₙ) angeordnet ist, die beiden Hydroformylierungsschritte also seriell (in Reihe) geschaltet sind. Dies bedeutet, dass die zweite Hydroformylierung als eine "Resteverwertung" der ersten Hydroformylierung vorgesehen ist und letztendlich die Oligomere der Cₙ₋₁ Alkene umsetzt, welche die erste Hydroformylierung nicht umgesetzt hat. Die Versorgung der zweiten Hydroformylierung mit Rohstoff geschieht folglich über die Einstellung des Grades des Teilumsatzes der ersten Hydroformylierung.

In einer Marktsituation, in der deutlich mehr C₉-Aldehyde nachgefragt werden als C₅₋ bzw. C₁₀-Aldehyde, muss bei dem in US2006/0122436A1 beschriebenen Verbundkonzept der Umsatz der ersten Hydroformylierung (die den C₅- und C₁₀-Markt bedient) sehr stark heruntergefahren werden, um für die zweite Hydroformylierung (die das begehrte C₉ macht) noch genügend nicht umgesetztes Buten übrig zu lassen. Dies führt dazu, dass die erste Hydroformylierung in einem sehr ungünstigen Betriebszustand gefahren werden muss und deswegen sehr ineffizient arbeitet.

Ein weiterer Nachteil der seriellen Verschaltung von Dehydrierung, erster Hydroformylierung, Oligomerisierung und zweiter Hydroformylierung rührt daher, dass kommerziell erhältliche Anlagen für die Dehydrierung von Alkanen, wie sie in Absatz [0022] der US2006/0122436A1 Erwähnung finden, in der Regel im Umfeld von Naphtha-Crackern betrieben werden, sodass sind diese Prozesse allesamt auf einen Durchsatz in petrochemischer Dimension ausgelegt und optimiert sind. So beträgt die Kapazität einer Propan-Dehydrierung nach dem STAR® Prozess etwa 500 000 t/a Propylen. Propan-Dehydrierungen nach dem CATOFIN®-Verfahren sind sogar für 850 000 t/a ausgelegt. Dies sind Größenordnungen, die sich sehr deutlich von denen der industriell betriebenen Hydroformylierung unterscheiden; so beträgt die Kapazität einer Oxo-Anlage typischerweise lediglich 100 000 t/a. Selbst wenn zwei große 250 kt/a-Oxo-Anlagen in der Lage wären, die von einer 500 kt/a Dehydrierung gelieferten Alkene zu verarbeiten, so müsste doch die erste Hydroformylierung zusätzlich überdimensioniert werden, um im Teillastfall eine entsprechend große Menge an nicht umgesetzten Alken für die zweite Oxo-Anlage durchschleifen zu können. Bei diesem Prozesslayout ist mithin die Dehydrierung zu groß bzw. sind die Hydroformylierungen zu klein, um als Gesamtprozess wirtschaftlich arbeiten zu können. Abhilfe würde hier nur eine kostspiele Spezialentwicklung einer ungewöhnlich kleinen Dehydrierung bieten oder aber die Möglichkeit, im Überschuss produziertes Alken anderweitig zu nutzen als für die AldehydHerstellung. Insoweit entstehen dann aber wieder neue Abnehmerabhängigkeiten.

Nach alledem besteht weiterhin der Bedarf ein Verfahren anzugeben, mit welchem sich sowohl C₅ - als auch C₉-Aldehyde wirtschaftlich produzieren lassen. Dabei soll das Verfahren mit möglichst geringer Abhängigkeit von Rohstofflieferanten versorgt werden können und zudem flexibel auf Nachfrageschwankungen hinsichtlich C₅ und C₉ Aldehyde reagieren können. Außerdem ist der Ressourceneinsatz des Verfahrens zu optimieren.

Gelöst wird diese Aufgabe durch ein Verfahren zur flexiblen Herstellung von Aldehyden mit fünf und neun Kohlenstoffatomen, welches die folgenden Schritte aufweist:
a) Bereitstellen von einem flüssigen Gemisch, genannt LPG bzw. NGL (Liquefied Petroleum Gas bzw. Natural Gas Liquids), welches genau eine Hauptkomponente, ausgewählt aus der Gruppe bestehend aus Propan, Isobutan und n-Butan, sowie mindestens eine Nebenkomponente ausgewählt aus der Gruppe bestehend aus Propan, Isobutan, n-Butan, Propen, Isobuten und n-Buten umfasst, unter der Maßgabe, dass die ausgewählte Hauptkomponente und die ausgewählte Nebenkomponente nicht identisch sind, und dass das Gemisch die folgende, sich zu 100 Gew.-% ergänzende, die Grenzwerte einschließende Zusammensetzung aufweist:

| | |
|---|---|
| • Propan: | 0 Gew.-% bis 50 Gew.%; |
| • Isobutan: | 0 Gew.-% bis 100 Gew.%; |
| • n-Butan: | 0 Gew.-% bis 100 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Summe sonstige Stoffe: | 0 Gew.-% bis 5 Gew.%; |

b) Abmischen eines Einsatzgemisches unter Verwendung des LPG bzw. des NGL;
c) für den Fall, dass das Einsatzgemisch mehr als 1.0 Gew.-% ungesättigte Kohlenwasserstoffe enthält: Reduzieren des Gehalts ungesättigter Kohlenwasserstoffe im Einsatzgemisch auf einen Wert unter 1.0 Gew.-% durch Unterwerfen des Einsatzgemisches einer Hydrierung;
d) optional: Reduzieren des Gehalts an n-Butan des Einsatzstoffgemisches durch Destillation des Einsatzgemisches unter Erhalt einer n-Butan enthaltenden Sumpffraktion, wobei der der Anteil an n-Butan in der n-Butan enthaltenden Sumpffraktion größer ist als der Anteil an n-Butan in dem destillierten Einsatzgemisch;
e) Dehydrieren des Einsatzgemisches unter Erhalt von mindestens einem Dehydrierungsgemisch,
f) Gewinnen einer C₄-Fraktion aus dem Dehydrierungsgemisch, wobei die C₄-Fraktion die folgende, sich zu 100 Gew.-% ergänzende, die Grenzwerte einschließende Zusammensetzung aufweist:

| | |
|---|---|
| • 1.3-Butadien: | 1 Gew.-% bis 5 Gew.-%; |
| • Isobuten: | 20 Gew.-% bis 50 Gew.-%; |
| • n-Buten: | 20 Gew.-% bis 50 Gew.-%; |
| • Summe Isobutan und n-Butan: | 2 Gew.-% bis 60 Gew.-%; |
| • Summe sonstige Stoffe: | 0 Gew.-% bis 1 Gew.-%; |

g) zumindest teilweises Entfernen von 1.3-Butadien und Isobuten aus der C₄-Fraktion unter Erhalt eines Intermediats, welches die folgende sich zu 100 Gew.-% ergänzende, die Grenzwerte einschließende Zusammensetzung aufweist:

| | |
|---|---|
| • 1.3-Butadien: | 0 Gew.-% bis 500 Gew.-ppm; |
| • Isobuten: | 0 Gew.-% bis 2 Gew.-%; |
| • n-Buten: | 30 Gew.-% bis 55 Gew.-%; |
| • Summe Isobutan und n-Butan: | 45 Gew.-% bis 70 Gew.-%; |
| • Summe sonstige Stoffe: | 0 Gew.-% bis 500 Gew.-ppm; |

h) Aufteilen des Intermediats in eine erste Portion und in eine zweite Portion in einem Splitter;
i) optional Reduzieren des Gehalts an Isobutan der ersten Portion des Intermediats durch Destillation der ersten Portion des Intermediats unter Erhalt einer Isobutan enthaltenden Sumpffraktion, wobei der der Anteil an Isobutan in der Isobutan enthaltenden Sumpffraktion größer ist als der Anteil an Isobutan in der destillierten ersten Portion des Intermediats und Verwenden der Isobutan enthaltenden Sumpffraktion zum Erhöhen des Gehalts an Isobutan des Einsatzgemisches, indem die Isobutan enthaltenden Sumpffraktion dem Einsatzgemisch zugegeben wird oder beim Abmischen des Einsatzgemisches verwendet wird;
j) Unterwerfen der ersten Portion des Intermediats einer Oligomerisierung in Gegenwart eines festen Katalysators, der amorphes Silicaalumina und mindestens 15 Gew.-% Nickel enthält, wodurch ein Oligomerisat erhalten wird, welches Olefine mit acht Kohlenstoffatomen und Butan enthält,
k) Abtrennen von Butan aus dem Oligomerisat und Verwenden des abgetrennten Butans beim Abmischen des Einsatzgemisches;
l) Abtrennen von Olefinen mit acht Kohlenstoffatomen aus dem Oligomerisat und Beaufschlagen der abgetrennten Olefinen mit acht Kohlenstoffatomen mit Synthesegas zwecks Durchführung einer ersten Hydroformylierung unter Erhalt eines ersten Hydroformylierungsgemisches, welches zumindest Aldehyde mit neun Kohlenstoffatome enthält;
m) Abtrennen einer ersten Zielfraktion enthaltend Aldehyde mit neun Kohlenstoffatomen aus dem ersten Hydroformylierungsgemisch;
n) Beaufschlagen der zweiten Portion des Intermediats mit Synthesegas zwecks Durchführung einer zweiten Hydroformylierung unter Erhalt eines zweiten Hydroformylierungsgemisches, welches zumindest Aldehyde mit fünf Kohlenstoffatome sowie Butan enthält;
o) Abtrennen von Butan aus dem zweiten Hydroformylierungsgemisch und Verwenden des abgetrennten Butans beim Abmischen des Einsatzgemisches;
p) Abtrennen einer zweiten Zielfraktion enthaltend Aldehyde mit fünf Kohlenstoffatomen aus dem zweiten Hydroformylierungsgemisch.

Ein solches Verfahren ist Gegenstand der Erfindung.

Das erfindungsgemäße Verfahren nutzt wahlweise LPG oder NGL als Rohstoff. Es kann somit über den offenen LPG-Markt aus vielen Quellen und von unterschiedlichen Lieferanten bedient werden. LPG wird auch in Tankschiffen transportiert und kann deswegen an Standorten mit Hafen in den benötigten Mengen angeliefert werden. Eine Abhängigkeit von einem einzelnen Crackern ist somit nicht mehr gegeben.

Alternativ kann das Verfahren mit NGL (Natural Gas Liquids) beschickt werden. NGL fällt auf einigen Erdgasfeldern insbesondere bei unkonventioneller Förderung an. NGL enthält Kohlenwasserstoffe mit zwei bis fünf Kohlenstoffatomen. LPG aus Erdgas ist somit als eine Teilfraktion von NGL aufzufassen. Der NGL-Markt ist heutzutage noch nicht so stark entwickelt wie der LPG-Markt, es werden aber in Zukunft Zuwächse erwartet:
Charles K. Ebinger und Govinda Avasarala: Natural Gas Liquids. https://www.brookings.edu/research/natural-gas-liquids-the-other-driver-of-the-u-s-oil-andgas-supply-resurgence/

Sowohl unter LPG als auch unter NGL ist im Sinne der Erfindung ein Gemisch zu verstehen, welches der folgenden Spezifikation gehorcht:

| | |
|---|---|
| • Propan: | 0 Gew.-% bis 50 Gew.%; |
| • Isobutan: | 0 Gew.-% bis 100 Gew.%; |
| • n-Butan: | 0 Gew.-% bis 100 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Summe sonstige Stoffe: | 0 Gew.-% bis 5 Gew.%; |

Selbstverständlich ergänzen sich die prozentualen Anteile der enthaltenen Stoffe zu 100 %. Die angegebenen Grenzen verstehen sich als Teil des Wertebereichs (mathematisch: geschlossenes Intervall). LPG bzw. NGL ist aber nie ein Reinstoff, sondern umfasst stets genau eine Hauptkomponente sowie eine Nebenkomponente oder mehrere Nebenkomponenten. Als Hauptkomponenten kommen nur Propan, Isobutan und n-Butan in Betracht. Nebenkomponente können alle oben gelisteten Stoffe sein mit Ausnahme der sonstigen Stoffe. Denknotwendig kann ein als Hauptkomponente gewählter Stoff nicht gleichzeitig eine Nebenkomponente sein.

Aus wirtschaftlicher Sicht bedeutend ist zudem das Merkmal, dass LPG durch Verflüssigung von Nebenprodukte entsteht, welche bei der Gewinnung von Erdöl bzw. Erdgas oder bei der Raffinierung von Rohöl anfallen, und dass es verflüssigt über eine eigene Lieferkette gehandelt wird.

Das erfindungsgemäße Verfahren kann wahlweise solche LPG-Typen verarbeiten, die vergleichsweise wenig oder vergleichsweise viel Propan enthalten. Das Prozesslayout unterscheidet sich dann in Details.

Ein an Propan armes LPG weist folgende Spezifikation auf:

| | |
|---|---|
| • Propan: | 0 Gew.-% bis 3 Gew.%; |
| • Isobutan: | 20 Gew.-% bis 80 Gew.%; |
| • n-Butan: | 20 Gew.-% bis 80 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Summe sonstige Stoffe: | 0 Gew.-% bis 5 Gew.%. |

Ein LPG-Typ, der viel Propan enthält, weist folgende Spezifikation auf:

| | |
|---|---|
| • Propan: | 10 Gew.-% bis 40 Gew.%; |
| • Isobutan: | 15 Gew.-% bis 85 Gew.%; |
| • n-Butan: | 15 Gew.-% bis 85 Gew.%; |
| • Propen: | 0 Gew.-% bis 3 Gew.%; |
| • Isobuten: | 3 Gew.-% bis 10 Gew.%; |
| • n-Buten: | 2 Gew.-% bis 15 Gew.%; |
| • Summe sonstige Stoffe: | 0 Gew.-% bis 5 Gew.%. |

Vorzugsweise wird ein Propanarmes LPG verwendet, dessen Hauptkomponente entsprechend Isobutan oder n-Butan ist.

Alternativ kann das Verfahren NGL (Natural Gas Liquids) als Rohstoff verarbeiten. NGL ist ein Gemisch aus Kohlenwasserstoffen mit zwei bis fünf Kohlenstoffatomen, es fällt bei der Förderung von Erdgas aus einigen Lagerstätten an, wenn der trockene Teil des Erdgases (das Methan) abgetrennt wird.

Typischerweise hat NGL die folgende, sich zu 100 Gew.-% ergänzende Zusammensetzung:

| | |
|---|---|
| • Ethan: | 0 Gew.-% bis 2 Gew.%; |
| • Propan: | 0 Gew.-% bis 50 Gew.%; |
| • Isobutan: | 0 Gew.-% bis 100 Gew.%; |
| • n-Butan: | 0 Gew.-% bis 100 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Pentan: | 0 Gew.-% bis 2 Gew.%; |
| • Summe sonstige Stoffe: | 0 Gew.-% bis 1 Gew.%. |

Vorteil von NGL ist, dass es unabhängig von Erdöl hergestellt wird.

Von dem aus US 2006/0122436 A1 bekannten, LPG basierten Verfahren unterscheidet sich das erfindungsgemäße Verfahren technologisch im Wesentlichen dadurch, dass das nach Dehydrierung und Abtrennung der Nebenprodukte erhaltene Intermediat vor der Hydroformylierung in zwei Portionen aufgeteilt wird. Aus der ersten Portion wird über Oligomerisierung und Hydroformylierung ein C₉-Aldehyd hergestellt, währenddessen das C₅-Aldehyd durch Hydroformylierung der zweiten Portion erhalten wird. Im Gegensatz dazu ist eine solche Aufteilung vor der Hydroformylierung in der US 2006/0122436 A1 nicht vorgesehen, sondern in bestimmten Fällen nur, dass eine Abtrennung der Cₙ-Aldehyde nach der (ersten) Hydroformylierung erfolgen kann und die restlichen Cₙ₋₁-Alkene in der Reihenfolge oligomerisiert, hydroformyliert und hydriert werden können, um C₂ₙ₋₁-Alkohole zu erhalten.

Die erste und zweite Hydroformylierung sind mithin erfindungsgemäß parallel angeordnet und nicht seriell. Dies hat den entscheidenden Vorteil, dass das Intermediat flexibel in die beiden Portionen aufgeteilt werden kann, sodass wahlweise mehr C₅ - oder mehr C₉ - Aldehyde herstellbar sind, abhängig von der jeweiligen Nachfrage.

Eine bevorzugte Ausführungsform des Verfahrens ist mithin dadurch gekennzeichnet, dass das Aufteilen des Intermediats in die erste Portion und in die zweite Portion unter Berücksichtigung eines sich zeitlich ändernden Bedarfs an Aldehyd mit neun Kohlenstoffatomen und an Aldehyd mit fünf Kohlenstoffatomen erfolgt, dergestalt, dass das Mengenverhältnis von erster Portion zu zweiter Portion sich mit der Zeit ändert analog zu der zeitlichen Änderung des Verhältnisses des Bedarfs an Aldehyd mit neun Kohlenstoffatomen zu dem Bedarf an Aldehyd mit fünf Kohlenstoffatomen.

Das Aufteilen der Portionen kann erfindungsgemäß in einem Splitter erfolgen, einer vergleichsweise einfachen Armatur, die das Intermediat mit einer einfachen Schieberbewegung mengenmäßig teilt, ohne dabei eine Stofftrennung vorzunehmen. Gegenüber der in US2006/0122436A1 betrieben Stofftrennung (i.e. Hydroformylierung von Cₙ₋₁ nach Cₙ) braucht somit nicht aufwändig der Umsatz der ersten Oxo-Anlage gesteuert werden und die C₄-Hydroformylierung muss bei hohem C₉-Bedarf auch keinen ineffizienten Betriebszustand einnehmen.

In einer nennenswerten Weiterbildung des Verfahrens wird die Oligomerisierung lediglich mit Teilumsatz gefahren. Der Anteil des Teilumsatzes kann zeitlich unveränderlich sein. Der Teilumsatz wirkt sich positiv auf die Isomerenverteilung im Oligomerisat aus, was insbesondere dann von Vorteil ist, wenn das der Oligomerisierung unterworfene Intermediat einen geringen Anteil von 1-Buten enthält. Dieser Effekt ist in WO2014/207034A1 eingehend geschildet.

Darüber hinaus erschließt der Teilumsatz auch einen weiteren Vorteil: Er macht es möglich, die Reaktionswärme der stark exothermen Oligomerisierung primär mit dem Oligomerisat aus der Reaktionszone abzuführen. Bevorzugt sollte mehr als 60% bis maximal 90 % der in der Oligomerisierung entstehenden Reaktionswärme mit dem Oligomerisat aus der Reaktionszone abgeführt werden. Da der Umsatz nicht vollständig ist, enthält das Oligomerisat noch genügend nicht umgesetztes Olefin, welches als Wärmeträger genutzt werden kann. Auch entsteht nicht allzu viel Reaktionswärme, weil die in die Reaktionszone eingebrachte Masse eben nur teilweise umgesetzt wird. Die Wärmeabfuhr über das Oligomerisat und der begrenzte Umsatz macht eine Wärmeabfuhr über ein externes Kühlmedium verzichtbar; die Investitions- und Betriebskosten des Reaktors werden dadurch reduziert. Oligomerisierung ohne externe Kühlung entspricht einer teiladiabaten Reaktionsführung.

Besonders bevorzugt wird sogar mehr als 90 % bis maximal 100 % der in der Oligomerisierung entstehenden Reaktionswärme mit dem Oligomerisat aus der Reaktionszone abgeführt. Dies entspricht dann einer adiabaten Reaktionsführung.

Gemäß Schritt g) des Verfahrens werden 1.3-Butadien und Isobuten vor der Hydroformylierung zumindest teilweise abgetrennt, sodass das Intermediat maximal 500 Gew.-ppm 1.3-Butadien und maximal 2 Gew.-% Isobuten enthält. Vorzugsweise werden diese beiden Substanzen vollständig abgetrennt, sodass das Intermediat frei von 1.3-Butadien und Isobuten ist. Grund für die Butadien-Abtrennung ist, dass 1.3 Butadien den Oligomerisierungskatalysator nachhaltig schädigt. Die Abtrennung von 1.3-Butadien erfolgt durch Selektivhydrierung, siehe auch DE102008007081A1, EP0820974B1 und US2006/0122436A1.

Isobuten wird entfernt, da dieses verzweigte Olefin es in der Hydroformylierung wiederum verzweigte Aldehyde bildet, was sich negativ auf die Produkteigenschaften der später aus den Aldehyden hergestellten Weichmacher auswirkt. Es ist somit zu erwarten, dass das vorliegende Verfahren eine andere Produktqualität liefert als das in US2006/0122436A1 beschriebene, weil dort das Isobuten nicht gesondert abgetrennt sondern explizit mit in die Hydroformylierung gefahren wird.

Die destillative Abtrennung von Isobuten aus einem Gemisch mit anderen C₄-Alkenen und C₄-Alkanen ist nicht trivial, da die Siedepunktunterschiede sehr gering sind. Dieses Problem wird dadurch gelöst, dass das Isobuten selektiv mit Methanol oder einem anderen Alkohol zu einem hochsiedenden Ether umgesetzt wird, der sich einfacher destillativ entfernen lässt. Eine bevorzugte Weiterbildung des Verfahrens sieht es deswegen vor, dass vor oder nach Durchführung der Selektivhydrierung das in der C₄-Fraktion enthaltende Isobuten mit einem Alkohol zumindest teilweise in einen entsprechenden Ether umgesetzt wird und der dabei gebildete Ether zumindest teilweise destillativ abgetrennt wird. Als Alkohol kommt vorzugsweise Methanol in Betracht, der mit Isobuten zu Methyl-tert.-butyl-Ether (MTBE) umgesetzt wird. Die Isobuten-Abtrennung über MTBE-Synthese hat sich industriell bewährt. Einzelheiten der MTBE-Technologie zur Abtrennung des Isobutens sind in DE102008007081A1 dargelegt.

Anstelle von Methanol kann auch Ethanol als Alkohol verwendet werden, der Ethyl-tert.-butyl-Ether (ETBE) bildet.

Die Isobuten-Abtrennung vor der Butadien-Abtrennung anzuordnen ist energetisch sinnvoll. Sofern der in der Ether-Synthese eingesetzte Katalysator jedoch empfindlich auf 1.3-Butadien reagiert, ist die Selektivhydrierung vor der Ether-Synthese anzuordnen.

Eine besondere Ausführungsform des Verfahrens sieht vor, dass es gar nicht ausschließlich mit LPG bzw. NGL gespeist wird, sondern dass beim Abmischen des Einsatzgemisches zusätzlich Isobutan und/oder n-Butan verwendet wird, welches weder aus dem bereitgestellten LPG bzw. NGL noch aus dem Oligomerisat oder aus dem zweiten Hydroformylierungsgemisch stammt.

Bei dem zur Rede stehenden zusätzlichen Isobutan und/oder n-Butan handelt es sich wie bei dem LPG bzw. NGL um einen externen Rohstoff, der von außen zugeführt wird. (Das Butan aus dem Oligomerisat und aus der zweiten Hydroformylierungsgemisch sind demgegenüber prozessinterne Kreislaufstoffe).

Dahinter steht die Idee, das vorliegende Verfahren zur "Resteverwertung" von sonst nicht weiter chemisch nutzbaren Isobutan und/oder n-Butan enthaltende Stoffströme zu nutzen.

Derartige Stoffströme bleiben am Ende der Verwertungskette eines herkömmlichen C₄-Stranges (wie in DE102008007081A1 offenbart) auf Grund der geringen Reaktivität der Alkane übrig und können deswegen nicht mehr chemisch genutzt werden. Sie werden stattdessen thermisch oder physikalisch genutzt, genauer gesagt verbrannt bzw. als Treibgas in Spraydosen eingesetzt.

Durch Einspeisen solcher Butanströme in den vorliegenden Prozess kann die Stoffeffizienz des Gesamtverbundes gesteigert werden, da eben diese Butane fortan chemisch genutzt, nämlich dehydriert werden.

Es sei in diesem Zusammenhang darauf hingewiesen, dass die Dehydrierung von Restalkanen ohne Zugabe von LPG bzw. NGL nicht wirtschaftlich ist, da die Dehydrierung prozesstechnisch sehr aufwendig und auch energieintensiv ist. Dazu kommen die bereits oben angesprochenen Größenordnungsunterschiede.

Die Idee, das vorliegende Verfahren zur Resteverwertung zu nutzen, beruht demnach auf der Erkenntnis, dass erst ein weiterer Rohstoff zugekauft werden muss (nämlich LPG bzw. NGL) um einen bereits vorhanden Reststoff (externes Isobutan und/oder n-Butan) wirtschaftlich verwerten zu können.

Je nach Verbundsituation kann es sogar möglich sein, beim Abmischen des Einsatzgemisches mengenmäßig mehr externes Butan zuzugeben als LPG bzw. NGL.

Die Technologie zur Dehydrierung von Alkanen unterscheidet oxidative Verfahren und nicht oxidative Verfahren. Bei der oxidativen Dehydrierung wird dem Alkangemisch ein Oxidationsmittel wie Sauerstoff oder Luft zugegeben, um den Wärmebedarf der stark endothermen Dehydrierung zumindest teilweise aus der Oxidation des frei gesetzten Wasserstoffs zu gewährleisten. Bei der nicht oxidativen Dehydrierung wird indes auf die Zugabe von Oxidationsmitteln verzichtet und stattdessen die benötigte Wärme von extern in den Reaktor eingebracht, etwa durch Beheizen mit einem Brenngas (meist Methan, Erdgas, Spaltgase aus dem Dehydrierprozess und ggf. teilweise Beimischung von Wasserstoff, der in der Dehydrierung gebildet wird). Beide Prozessvarianten unterscheiden sich stark in der Zusammensetzung des Dehydrierungsgemisches. Eine detaillierte Abhandlung über die gängige Dehydrierungstechnologie findet sich in US2006/0122436A1.

Diese Schrift empfiehlt die oxidative Dehydrierung. Demgegenüber ist es hier bevorzugt, dass die Dehydrierung zumindest abschnittsweise ohne Zugabe eines Oxidationsmittels - also nicht oxidativ - erfolgt. Grund dafür ist, dass die nicht oxidative Dehydrierung selektiver ist, zudem kann der frei gesetzte Wasserstoff nach Abtrennung und Aufreinigung beispielsweise über eine Druckwechselabsorption auch für die im Verfahren vorgesehenen Hydrierungen eingesetzt werden. Bei einer oxidativen Dehydrierung wird der freigesetzte Wasserstoff unmittelbar wieder verbrannt, mit dem Ziel dabei das Gleichgewicht nach LeChatelier zu den gebildeten Olefinen hin zu verschieben.

Die Formulierung "zumindest abschnittsweise ohne Zugabe eines Oxidationsmittels" berücksichtigt den Umstand, dass einige kommerziell erhältliche, nicht oxidative Dehydrierungen am Beginn ihrer Reaktionszone bzw. unmittelbar davor eine Wasserstoff-Einspeisung vorsehen.

Die prozesstechnisch einfachste Möglichkeit, die Dehydrierung in das Verfahren einzugliedern besteht darin, die C₃- und C₄-Alkane gleichzeitig und am selben Ort zu dehydrieren. Die entsprechende Ausführungsform des Verfahrens sieht es demnach vor, dass die Dehydrierung des Einsatzgemisches in einer Reaktionszone erfolgt, und dass die im Einsatzgemisch enthaltenden Alkane mit drei und vier Kohlenstoffatomen gemeinsam in derselben Reaktionszone dehydriert werden.

Die "Reaktionszone" ist in diesem Zusammenhang der Ort, an dem die Dehydrierung stattfindet. Im einfachsten Fall ist das genau ein Reaktor. Es können aber auch mehrere Reaktoren vorgesehen sein, die parallel oder seriell verschaltet sind. Die Gesamtheit dieser so verschalteten Reaktoren bildet dann die Reaktionszone. Bei der gemeinsamen Dehydrierung von Propan und Butan in derselben Reaktionszone werden beide Alkane bei denselben Reaktionsbedingungen und an demselben Katalysator dehydriert.

Dies setzt natürlich voraus, dass die Dehydrierung der C₃ und C₄ Alkane gleichzeitig an demselben Katalysator gelingt. Dies ist in der Regel der Fall, jedoch ist die Effizienz der Misch-Dehydrierung begrenzt und es entstehen mehr unerwünschte Nebenprodukte. Effizienter ist es daher, Propan und Butan getrennt zu dehydrieren.

Die zugehörige Ausführungsform des Verfahrens sieht demnach vor, dass die Dehydrierung des Einsatzgemisches in mindestens zwei Reaktionszonen erfolgt, wobei im Einsatzgemisch enthaltenden Alkane mit drei Kohlenstoffatomen in der ersten Reaktionszone dehydriert werden, und wobei im Einsatzgemisch enthaltenden Alkane mit vier Kohlenstoffatomen in der zweiten Reaktionszone dehydriert werden.

Prozesstechnisch ist die getrennte Dehydrierung in dedizierten Reaktionszonen aufwendiger als eine Misch-Dehydrierung, aber stofflich effizienter. Im Einzelfall entscheidet sich, welche Variante die wirtschaftlichere ist. Ein wichtiges Entscheidungskriterium wird die Zusammensetzung des angelieferten LPG sein: Sofern die Anlage ausschließlich mit C₃-armen LPG beschickt wird, macht die Installation dedizierter Reaktionszonen keinen Sinn.

Prinzipiell ist es auch denkbar eine Variante zu wählen, in der drei dedizierte Reaktionszonen betrieben werden, eine erste für Propan, eine zweite für n-Butan und eine dritte für Isobutan.

Zudem ist es auch möglich eine erste Reaktionszone für die Misch-Dehydrierung für Isobutan und Propan vorzusehen und eine weitere Reaktionszone für die dedizierte Dehydrierung von n-Butan. Dies macht insbesondere dann Sinn, wenn eine Kolonne zur Trennung von n-Butan und Isobutan installiert wird. Mit einer solchen Kolonne wird der optionale Verfahrensschritt d), also die Reduzierung des Gehalts an n-Butan des Einsatzstoffgemisches, ausgeführt. An dessen Sumpf fällt eine Schwersieder-Fraktion an, die besonders reich an n-Butan ist. Sofern keine andere Verwertungsmöglichkeit für diese Schwersieder besteht, kann die Sumpffraktion der Kolonne zum Trennen von n-Butan und Isobutan teilweise oder vollständig in eine weitere Reaktionszone für Dehydrierung von n-Butan eingebracht werden. Die entsprechende Verfahrensvariante ist mithin dadurch gekennzeichnet, dass eine weitere Reaktionszone vorgesehen ist, in welcher die Dehydrierung des destillierten Einsatzgemisches nicht erfolgt, wohingegen die n-Buten enthaltende Sumpffraktion in der weiteren Reaktionszone einer Dehydrierung unterworfen wird. Diese Verfahrensvariante setzt die Ausführung des Schrittes d) voraus.

Bleibt zu erwähnen, dass die dedizierte Dehydrierung im begrenzten Umfang immer eine Misch-Dehydrierung darstellen wird, da die Destillationskolonnen, welche die Fraktionierung der einzelnen Alkane vornehmen, aus wirtschaftlichen Gründen nicht mit der technisch möglichen Trennschärfe gefahren werden sollten. Die Propan-Fraktion, die in der ersten Reaktionszone dehydriert wird, kann deswegen durchaus auch geringfügige Restmengen an Butan enthalten. Umgekehrt kann die dedizierte Butan-Dehydrierung auch in Gegenwart von Restmengen an Propan erfolgen.

Verfahrensschritt f) - also die Gewinnung der C₄-Fraktion aus dem Dehydrierungsgemisch - erfolgt einfachstenfalls mit Hilfe einer Destillationskolonne, bei der die Leicht siedenden Bestandteile des Dehydrierungsgemisches (C₁- bis C₃-Kohlenwasserstoffe, H₂, CO₂) über Kopf abgenommen und die C₄-Fraktion vom Sumpf abgezogen wird.

Die entsprechende Verfahrensvariante ist dadurch gekennzeichnet, das das Gewinnen der C₄-Fraktion aus dem Dehydrierungsgemisch bzw. den Dehydrierungsgemischen dadurch erfolgt, dass das Dehydrierungsgemisch bzw. die Dehydrierungsgemische gemeinsam destilliert werden, wobei die C₄-Fraktion als Schwersieder zurück bleiben und mindestens eine Leichtsiederfraktion gewonnen wird.

Der Vorteil dieser Vorgehensweise ist, dass die Leichtsieder am Kopf gasförmig anfallen und sodann auch gasförmig genutzt werden können, nämlich als Brenngas zum Beheizen der Dehydrierung.

Sofern mehrere dedizierte Reaktionszonen vorgesehen sind, fallen daraus auch entsprechend mehrere Dehydrierungsgemische an. Die Leichtsieder dieser Dehydrierungsgemische können gemeinsam in derselben Kolonne von der C₄-Fraktion getrennt werden, da außerhalb der Reaktionszonen keine unerwünschten Folgereaktionen mehr zu erwarten sind. Die gemeinsame Aufarbeitung der Dehydrierungsgemische senkt mithin die Apparatekosten.

Einige Verfahrensvarianten sollen nun anhand von vereinfachten Prozessfließbildern erläutert werden. Es zeigen:
- Figur 1:: Basisausführung des Verfahrens;
- Figur 2:: Verfahrensvariante mit Kolonne zum Trennen von n-Butan und Isobutan sowie mit dedizierter C₃- und C₄- Dehydrierung;
- Figur 3:: Verfahrensvariante mit Kolonne zum Trennen von n-Butan und Isobutan, mit dedizierter n-Butan-Dehydrierung sowie Misch-Dehydrierung von Propan und Isobutan;
- Figur 4:: Verfahrensvariante mit Kolonne zum Trennen von n-Butan und Isobutan sowie mit dedizierter Dehydrierung von Propan, Isobutan und n-Butan;
- Figur 5:: Verfahrensvariante mit Kolonne zum Trennen von n-Butan und Isobutan sowie mit Misch-Dehydrierung und interner Isobutan-Rückführung;

Eingangs wird ein Einsatzgemisch 1 abgemischt. Hierzu wird LPG mit einem im Wesentlichen Butan enthaltenden Rücklauf 2 aus dem Prozess vermischt. Je nach Zusammensetzung des verwendeten LPG enthält das Einsatzgemisch 1 im Wesentlichen C₃- und/oder C₄-Alkane. Darüber hinaus können auch nennenswerte Mengen an C₃- und C₄ - Olefine enthalten sein. Anstelle von LPG kann auch NGL verwendet werden, dann sind auch C₂ -und C₅-Kohlenwasserstoffe enthalten.

Für den Fall, dass das Einsatzgemisch 1 mehr als 1.0 Gew.-% ungesättigte Kohlenwasserstoffe wie Alkene oder Alkine enthält, muss der Gehalts an ungesättigten Kohlenwasserstoffen im Einsatzgemisch 1 reduziert werden auf einen Wert unter 1.0 Gew.-%. Grund dafür ist die Empfindlichkeit der nachfolgenden Dehydrierung gegen Olefine, denn diese bewirken rasch eine Verkokung des Katalysators. Um die ungesättigten Verbindungen zu beseitigen wird das Einsatzgemisch 1 einer Hydrierung 3 unterworfen, bei der an einem heterogenen Katalysator die ungesättigten Verbindungen durch Zugabe von Wasserstoff H₂ gesättigt werden. Aus den Olefinen entstehen so die entsprechenden Alkane.

Zur Hydrierung kann ein beliebiger Katalysator eingesetzt werden, der in der Lage ist Olefine zu hydrieren. Gängige kommerzielle Katalysatoren verwenden hier beispielsweise Pd, Pt oder Ni als Aktivkomponente, die zumeist auf einen Träger aus Al₂O₃ oder SiO₂ aufgebracht sind. Es können auch weitere Komponenten enthalten sein. Es gibt auch Mischsysteme mit Pd und Pt als Aktivkomponente. Die Trägersysteme können auch Mischungen aus Al₂O₃ zusammen mit SiO₂ sein. Die Hydrierung erfolgt bei erhöhtem Druck unter Zugabe von Wasserstoff in der Regel in einem Temperaturbereich zwischen 20°C und 200 °C. Die Hydrierung kann in der Flüssigphase oder der Gasphase stattfinden, wobei der Phasenzustand sich aus der Regelung von Druck und Temperatur ergibt. Hydrierung in der Flüssigphase ist vorteilhaft, da das LPG flüssig angeliefert wird.

Das Einsatzgemisch 1 gelangt nun in eine Destillationskolonne 4 zum Trennen von n-Butan und Isobutan. Im vorliegenden Prozess eingesetzt reduziert diese Kolonne 4 den Gehalt an n-Butan im Einsatzgemisch 1; es reichert sich in dessen Sumpffraktion 5 an. Diese enthält neben n-Butan auch etwaige weitere Kohlenwasserstoffe mit mehr als vier Kohlenstoffatomen C₄₊. Die Sumpffraktion 5 wird aus dem Prozess ausgeschleust (purge). Die leichter siedenden Bestandteile, vor allem Propan und Isobutan, gehen über Kopf. Damit die Investitions- und Betriebskosten der Kolonne zum Trennen von n-Butan und Isobutan 4 nicht zu hoch ausfallen, wird sie weniger scharf gefahren, sodass auch n-Butan über Kopf geht. Grundsätzlich sollte aber der Anteil an n-Butan in der Sumpffraktion 5 der Kolonne 4 zum Trennen von n-Butan und Isobutan größer sein als der Anteil an n-Butan in dem destillierten Einsatzgemisch 1, welches vom Kopf der Kolonne 4 abgezogen wird. Falls das Einsatzgemisch kaum n-Butan enthält, ist Kolonne 4 verzichtbar.

Das Einsatzgemisch 1 wird nun einer Dehydrierung unterworfen. Dafür ist eine Reaktionszone 6 eingerichtet, die von einem Reaktor gebildet wird. Der Reaktor enthält einen heterogenen Katalysator und wird mit einem Brenngas beheizt (nicht dargestellt). Alternativ kann auch eine oxidative Dehydrierung vorgesehen sein, deren Wärmebedarf durch Verbrennung eines Teils des Einsatzgemisches deckt.

In beiden Fällen wird aus der Reaktionszone ein Dehydrierungsgemisch 7 abgezogen. Dessen Zusammensetzung hängt stark von dem Einsatzgemisch und den Dehydrierungsbedingungen ab. Da bei der Dehydrierung die gesättigten Bindungen in ungesättigte überführt werden (umgekehrt wie bei der Hydrierung) enthält das Dehydrierungsgemisch in jedem Fall die zu den im Einsatzgemisch enthaltenen Alkane korrespondierende Alkene. Es können aber auch mehrfach ungesättigte Verbindungen enthalten sein sowie Nebenprodukte und vor allem der frei werdende Wasserstoff.

Aus dem Dehydrierungsgemisch 7 wird nun eine C₄-Fraktion C₄ abgetrennt. Die C₄-Fraktion setzt sich wie folgt zusammen:

| | |
|---|---|
| • 1.3-Butadien: | 1 Gew.-% bis 5 Gew.-%; |
| • Isobuten: | 20 Gew.-% bis 50 Gew.-%; |
| • n-Buten: | 20 Gew.-% bis 50 Gew.-%; |
| • Summe Isobutan und n-Butan: | 2 Gew.-% bis 60 Gew.-%; |
| • Summe sonstige Stoffe: | 0 Gew.-% bis 1 Gew.-%; |

Das Gewinnen der C₄-Fraktion C₄ aus dem Dehydrierungsgemisch 7 erfolgt dadurch, dass das Dehydrierungsgemisch 7 destilliert wird, wobei die C₄-Fraktion die Schwersieder bildet und eine Leichtsiederfraktion 9 gewonnen wird. Die Leichtsiederfraktion 9 enthält im Wesentlichen Kohlenwasserstoffe mit ein bis drei Kohlenstoffatome, Wasserstoff und Kohlendioxid. Die Leichtsiederfraktion 9 wird als Brenngas zur Beheizung der Reaktionszone 6 genutzt (nicht dargestellt). Das Kopfprodukt der zweiten Destillationskolonne 8 braucht deswegen nicht kondensiert werden.

Sofern die C₄-Fraktion mehrfach ungesättigte Verbindungen wie insbesondere 1.3-Butadien enthält, sind diese nun zu entfernen, da sie nachfolgende Katalytische Prozesse vergiften können, insbesondere die Oligomerisierung.

Eine einfache Hydrierung ist jedoch nicht möglich, da so die erst eben hergestellten Butene wieder verloren gingen. Deswegen wird die C₄-Fraktion einer so genannten Selektivhydrierung 10 unterworfen, die das 1.3-Butadien selektiv umsetzt, die Butene jedoch nicht hydriert. Dies geschieht mit speziellen Katalysatoren und Zugabe von CO als Moderator.

Nun gilt es, aus der C₄-Fraktion C₄ das Isobuten zu entfernen. Dies ist destillativ nicht möglich aufgrund des geringen Siedepunktunterschieds gegenüber 1-Buten. Deswegen ist eine MTBE-Synthese 11 vorgesehen, in welcher das Isobuten mit Methanol MeOH selektiv zu Methyl.-tert. Butylether (MTBE) umgesetzt wird. Das hoch siedende MTBE kann nun vom Sumpf einer dritten Destillationskolonne 12 abgezogen und ausgeschleust werden. Es eignet sich vor allem als Kraftstoffzusatz, kann aber auch in hochreines Isobuten zurückgespalten werden oder nach weiterer Aufreinigung als Lösemittel verwendet werden. Alternativ zu MTBE kann die Abtrennung auch als ETBE erfolgen, dazu wird Ethanol statt Methanol als Alkohol verwendet.

Sofern der in der MTBE-Synthese 11 eingesetzte Katalysator unempfindlich auf mehrfach ungesättigte Kohlenwasserstoffe reagiert, kann die Selektivhydrierung 10 auch stromabwärts hinter der MTBE-Synthese 11 angeordnet werden.

Am Kopf der dritten Destillationskolonne 12 wird ein Intermediat 13 abgegriffen. Dabei handelt es sich um ein Gemisch aus C₄-Kohlenwasserstoffen mit folgender Spezifikation:

| | |
|---|---|
| • 1.3-Butadien: | 0 Gew.-% bis 500 Gew.-ppm; |
| • Isobuten: | 0 Gew.-% bis 2 Gew.-%; |
| • n-Buten: | 30 Gew.-% bis 55 Gew.-%; |
| • Summe Isobutan und n-Butan: | 45 Gew.-% bis 70 Gew.-%; |
| • Summe sonstige Stoffe: | 0 Gew.-% bis 500 Gew.-ppm; |

Den Wertbestandteil bilden die linearen C₄-Olefine 1-Buten, cis-2-Buten und trans-2-Buten, die als n-Buten zusammen gefasst sind. Gleichwohl ist weiterhin ein großer Anteil reaktionsträger C₄-Akane enthalten. Die Summe von Isobutan und n-Butan kann sogar den Anteil der linearen Butene übersteigen. Verglichen mit anderen C₄-Rohstoffströmen wie Crack-C₄ oder FCC-C₄ ist das Intermediat 13 auffällig stark mit inerten Alkanen verdünnt. Störstoffe sind hingegen kaum mehr enthalten.

Gemäß der Erfindung wird das Intermediat 13 nun in zwei Portionen 14, 15 aufgeteilt. Dies geschieht in einem Splitter 16, einer einfachen Armatur zum mengenmäßigen Teilen ohne Stofftrennung. Die stoffliche Zusammensetzung von erster Portion 14, zweiter Portion 15 und Intermediat 13 ist demnach identisch.

Die Mengenaufteilung zwischen erster und zweiter Portion erfolgt flexibel nach der derzeitigen Bedarfslage: Wird viel C₉-Aldehyd benötigt (etwa für die Herstellung des Weichmacheralkohols Isononanol) wird der Splitter 16 so eingestellt, dass die erste Portion 14 größer ist als die zweite Portion 15. Wenn dagegen mehr C₅ - bzw. C₁₀ -Aldehyd benötigt wird (Herstellung von Valeraldehyd bzw. des Weichmacheralkohols 2-Propylheptanol) wird die zweite Portion 15 gegenüber der ersten Portion 14 vergrößert.

Grund für die flexible, bedarfsorientierte Mengenaufteilung des Intermediats 13 in seine beiden Portionen 14 und 15 ist, dass sich die Produktionskette an dem Splitter 16 in zwei parallele Stränge aufteilt: Der erste Strang wird mit der ersten Portion 14 und der zweite Strang mit der zweiten Portion 15 des Intermediats 13 gespeist. Der erste Strang dient zur Herstellung von C₉-Aldehyd, währenddessen der zweite Strang für die C₅ - bzw. C₁₀ - Herstellung bestimmt ist.

Für die C₉-Herstellung ist erst einmal eine Oligomerisierung der C₄ -Olefine zu C₈ - Olefinen erforderlich. Dafür wird die erste Portion 13 einer Oligomerisierung 17 unterworfen. Diese erfolgt in Gegenwart eines festen Katalysators, der amorphes Silicaalumina und mindestens 15 Gew.-% Nickel enthält. Der angegebene Nickelgehalt bezieht sich auf elementares Nickel. Als Nickeloxidgerechnet, entspricht das etwa 20 Gew.-%. Ein geeigneter Katalysator und dessen Herstellung ist in US2581228 offenbart. Die Oligomerisierung erfolgt weitestgehend adiabat, das heißt ohne Wärmeaustausch mit der Umgebung über ein externes Kühlmittel. Die Reaktionswärme wird mit dem Oligomerisat 18 ausgetragen.

Das Oligomerisat 18 ist das Produkt der Oligomerisierung und enthält zumindest Olefine mit acht Kohlenstoffatomen (Dimere des Butens) sowie n-Butan und/oder Isobutan, da diese Stoffe sich in der Oligomerisierung 17 inert verhalten. Zudem enthält das Oligomerisat höhere Oligomere des Butens, etwa Trimere (C₁₂-Olefine) und Tetramere (C₁₆-Olefine) des n-Butens.

Die in dem Oligomerisat 18 enthaltenden Butane sowie nicht umgesetzte restliche Butene, bevorzugt mit einem Anteil von weniger als 10 Gew.-%, werden nun in einer vierten Kolonne 19 über Kopf abgetrennt und als Rücklauf 2 bei der Abmischen des Einsatzgemischs 1 wiederverwertet. Die Butane gelangen so wieder in die Dehydrierung und werden dort stofflich verwertet.

Vom Sumpf der vierten Destillationskolonne werden die im Oligomerisat 18 enthaltenden Kohlenwasserstoffe mit mehr als vier Kohlenstoffatome C₄₊ abgezogen. Das sind fast ausschließlich Olefine. Diese werden in einer fünften Destillationskolonne 20 weiter aufgeteilt in die Olefine mit acht Kohlenstoffatome C₈ und Olefine mit mehr als acht Kohlenstoffatomen C₈₊. Letzte werden vom Sumpf der fünften Destillationskolonne 20 abgezogen und ausgeschleust.

Die am Kopf der fünften Destillationskolonne 20 gewonnenen Olefine mit acht Kohlenstoffatomen C₈ werden nun zusammen mit Synthesegas (Syngas, normalerweise eine 1:1 Mischung aus Wasserstoff und Kohlenmonoxid) in einer ersten Hydroformylierung 21 zu Aldehyd mit neun Kohlenstoffatomen umgesetzt. Diese finden sich in einem ersten Hydroformylierungsgemisch 22, welches aus der ersten Hydroformylierung 21 abgezogen wird. Da bei der Hydroformylierung auch hochsiedende Nebenprodukte C₉₊ entstehen und diese im ersten Hydroformylierungsgemisch 22 enthalten sind, muss das erste Hydroformylierungsgemisch 22 noch aufgearbeitet werden. Die Aufarbeitung findet in zumindest einer sechsten Destillationskolonne 23 statt. An dessen Kopf wird eine erste Zielfraktion C₉ gewonnen, die den gewünschten Aldehyd mit neun Kohlenstoffatomen enthält. Die Hochsieder C₉₊ bleiben im Sumpf der sechsten Destillationskolonne 23 und werden ausgeschleust.

Einzelheiten zur C₈-Hydroformylierung und zur anschließenden Produktabtrennung sind WO2014/131623A1 entnehmen.

Die zweite Portion 15 des Intermediats 13 wird indes einer zweiten Hydroformylierung 24 unterworfen, bei der aus dem enthaltenden n-Buten Aldehyde mit fünf Kohlenstoffatome gebildet. Es wird ein zweites Hydroformylierungsgemisch 25 erhalten, in dem neben den gewünschten Pentanalen auch hochsiedende Nebenprodukte C₅₊ enthalten sind.

In einer siebten Destillationskolonne 26 findet die Abtrennung einer zweiten Zielfraktion C₅ enthaltend die Aldehyde mit fünf Kohlenstoffatomen aus dem zweiten Hydroformylierungsgemisch 25 statt. Die siebten Destillationskolonne 26 ist hier als Seitenabzugskolonne ausgeführt und die zweite Zielfraktion C₅ wird an dem Seitenabzug abgenommen. Am Kopf werden die in der Hydroformylierung 24 nicht umsetzbaren Butane abgezogen und als Rücklauf 2 beim Bereitstellen des Einsatzgemisches 1 wiederverwertet. Die Hochsieder C₅₊ bleiben im Sumpf der siebten Destillationskolonne 26 und werden ausgeschleust. Die C₅-Aldehyde können anschließend noch mittels Aldolkondensation zu C₁₀ Aldehyden weiterverarbeitet werden (nicht dargestellt).

Einzelheiten zur C₄-Hydroformylierung sind der WO2014/056732A1 zu entnehmen. Darin finden sich auch Querverweise zur anschließenden Produktabtrennung und zur Aldolkondensation.

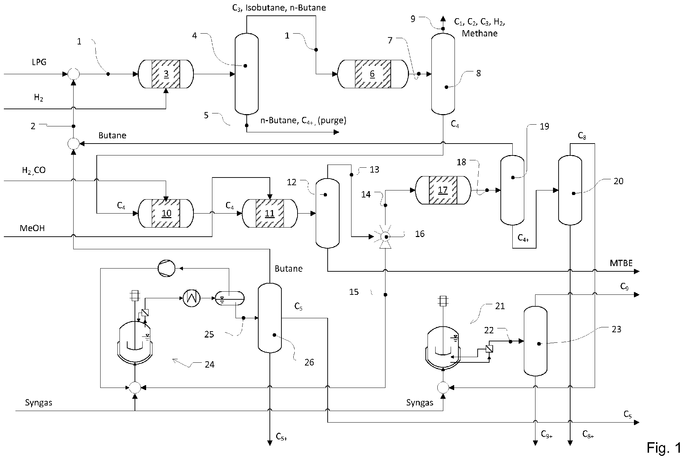

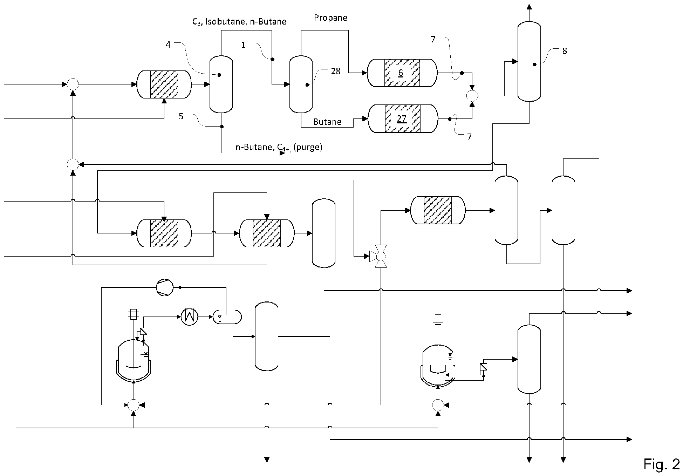

Die in Figur 2 dargestellte Verfahrensvariante unterscheidet sich von der in Figur 1 gezeigten Basisvariante dadurch, dass eine dedizierte C₃ und C₄-Dehydrierung erfolgt. Für diesen Zweck ist die erste Reaktionszone 6 einzig der Propan-Dehydrierung gewidmet, für die Dehydrierung von C₄-Alkanen ist eine zweite Reaktionszone 27 vorgesehen. Die in den Reaktionszonen 6, 27 herrschenden Betriebsbedingungen sowie die dort angeordneten Katalysatoren sind unterschiedlich.

Um das Einsatzgemisch 1 auf die beiden Reaktionszonen aufzuteilen ist direkt hinter der Kolonne 4 zum Trennen von n-Butan und Isobutan eine achte Destillationskolonne 28 angeordnet, welche mit dem Kopfprodukt der Kolonne 4 (i.e. destilliertes Einsatzgemisch) gespeist wird. Die achte Destillationskolonne 28 nimmt eine Propan/Butan-Trennung vor. Propan geht über Kopf in die erste Reaktionszone 6 und Butan aus dem Sumpf der achten Destillationskolonne 28 in die zweite Reaktionszone 27.

Die aus den beiden Reaktionszonen 6, 27 jeweils abgezogenen Dehydrierungsgemische werden vereinigt und gemeinsam in der zweiten Destillationskolonne 8 aufgearbeitet.

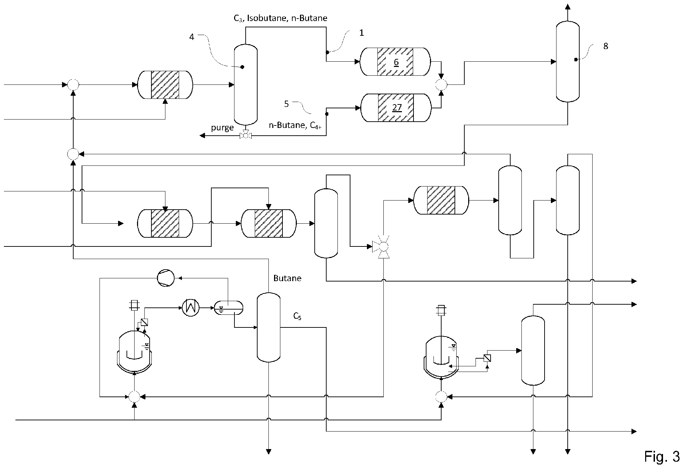

Die in Figur 3 gezeigte Prozessvariante unterscheidet sich von der in Figur 1 gezeigten Basisausführung dadurch, dass die an n-Butan reiche Sumpffraktion 5 der Kolonne 4 zum Trennen von n-Butan und Isobutan teilweise ausgeschleust und teilweise in eine dedizierte Dehydrierung gefahren wird, die in einer zweiten Reaktionszone 27 erfolgt. Wie bei Figur 2 werden die beiden Dehydrierungsgemische gemeinsam weiter aufgearbeitet. Gegenüber dem in Figur 2 gezeigten Prozess wurde auf eine Propan/Butan-Trennung zwischen Kolonne 4 und Dehydrierung 6, 27 verzichtet. Dafür findet in der ersten Reaktionszone 6 eine Misch-Dehydrierung statt.

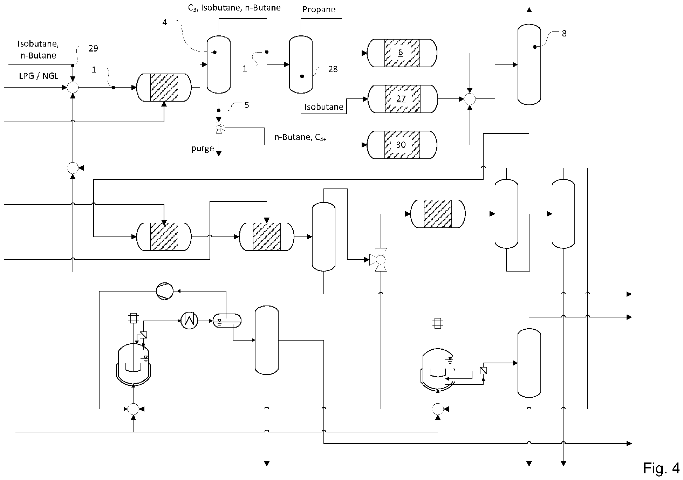

Ein wichtiges Merkmal des in Figur 4 gezeigten Prozesses besteht darin, dass dieser neben LPG bzw. NGL noch eine zweite Rohstoffquelle nutzt. Dabei handelt es sich um Isobutan und/oder n-Butan, welches in einem Strom 29 von extern zugeführt wird. Es kann sich dabei um Reststoffe aus einer weiteren, hier nicht dargestellten Hydroformylierung handeln. Dieser Strom wird mit dem LPG bzw. NGL und dem Butan aus dem Rücklauf 2 zu dem Einsatzgemisch 1 abgemischt.

Der in Figur 4 gezeigte Prozess weist drei dedizierte Dehydrierungen auf: In der ersten Reaktionszone 6 wird Propan dehydriert, in der zweiten Reaktionszone 27 vornehmlich Isobutan und etwas n-Butan und in der dritten Reaktionszone 30 im Wesentlichen n-Butan. Zur Aufteilung des Einsatzgemisches 1 auf die drei Reaktionszonen 6, 27, 30 sind zwei Kolonnen erforderlich, nämlich eine Kolonne 4 zum Trennen von n-Butan und Isobutan sowie eine achte Destillationskolonne 28 zur Propan/Butan-Trennung. Die Dehydrierungsgemische werden gemeinsam aufgearbeitet.

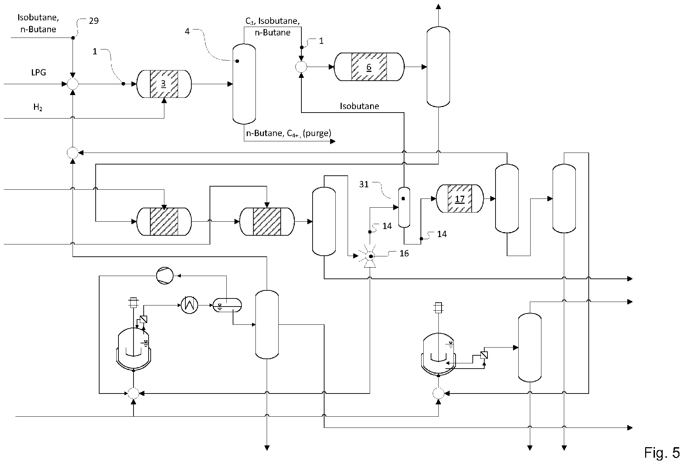

Für den in Figur 5 angenommen Fall, dass das Einsatzgemisch 1 sehr viel Isobutan enthält (etwa wegen einer entsprechenden LPG-Qualität oder einem hohen Gehalt an Isobutan in dem Strom 29 aus der zweiten Rohstoffquelle) empfiehlt es sich, vor der Oligomerisierung 17 zumindest einen großen Teil des Isobutans abzudestillieren, um die Konzentration der n-Butene zu erhöhen und die Kinetik der Oligomerisierung zu nutzen, um höhere Co-Olefinausbeuten zu erhalten.

Für diesen Zweck ist in der in Figur 5 eine neunte Destillationskolonne 31 vorgesehen, welche das Isobutan über Kopf aus der ersten Portion 13 des Intermediats abtrennt. Es wird zusammen mit dem Einsatzgemisch 1 in die Reaktionszone 6 gefahren wird, wo die Dehydrierung stattfindet. Es empfiehlt sich, das Isobutan erst hinter der Kolonne 4 mit dem Einsatzgemisch 1 zu vermischen, sodass es nicht unnötig durch die Hydrierung 3 geschleift und nochmal in Kolonne 4 verdampft werden muss. Im Übrigen entspricht dieses Prozesslayout der in Figur 1 gezeigten Basisausführung.

### Bezugszeichenliste

- 1: Einsatzgemisch
- 2: Rücklauf (Butan enthaltend)
- 3: Hydrierung
- 4: Kolonne zum Trennen von n-Butan und Isobutan
- 5: Sumpffraktion
- 6: Reaktionszone
- 7: Dehydrierungsgemisch
- 8: zweite Destillationskolonne
- 9: Leichtsiederfraktion
- 10: Selektivhydrierung
- 11: MTBE-Synthese
- 12: dritte Destillationskolonne
- 13: Intermediat
- 14: erste Portion des Intermediats
- 15: zweite Portion des Intermediats
- 16: Splitter
- 17: Oligomerisierung
- 18: Oligomerisat
- 19: vierte Destillationskolonne
- 20: fünfte Destillationskolonne
- 21: erste Hydroformylierung (von C₈ nach C₉)
- 22: erstes Hydroformylierungsgemisch
- 23: sechste Destillationskolonne
- 24: zweite Hydroformylierung (von C₄ nach C₅)
- 25: zweites Hydroformylierungsgemisch
- 26: siebte Destillationskolonne
- 27: zweite Reaktionszone
- 28: achte Destillationskolonne
- 29: Strom enthaltend n-Butan und Isobutan
- 30: dritte Reaktionszone
- 31: neunte Destillationskolonne
- C₄: C₄-Fraktion
- C₄₊: Kohlenwasserstoffe mit mehr als vier C-Atomen
- C₅: Aldehyde mit fünf Kohlenstoffatomen (zweite Zielfraktion)
- C₅₊: Hochsieder
- C₈: Olefine mit acht Kohlenstoffatomen
- C₈₊: Olefine mit mehr als acht Kohlenstoffatomen
- C₉: Aldehyde mit neun Kohlenstoffatomen (erste Zielfraktion)
- C₉₊: Hochsieder
- MeOH: Methanol
- MTBE: Methyl-tert. Butylether
- Syngas: Synthesegas

## Patentansprüche

1. Verfahren zur flexiblen Herstellung von Aldehyden mit fünf und neun Kohlenstoffatomen, welches die folgenden Schritte aufweist:
a) Bereitstellen von einem flüssigen Gemisch, genannt LPG bzw. NGL (Liquefied Petroleum Gas bzw. Natural Gas Liquids), welches genau eine Hauptkomponente, ausgewählt aus der Gruppe bestehend aus Propan, Isobutan und n-Butan, sowie mindestens eine Nebenkomponente ausgewählt aus der Gruppe bestehend aus Propan, Isobutan, n-Butan, Propen, Isobuten und n-Buten umfasst, unter der Maßgabe, dass die ausgewählte Hauptkomponente und die ausgewählte Nebenkomponente nicht identisch sind, und dass das Gemisch die folgende, sich zu 100 Gew.-% ergänzende, die Grenzwerte einschließende Zusammensetzung aufweist:
| | |
|---|---|
| • Propan: | 0 Gew.-% bis 50 Gew.%; |
| • Isobutan: | 0 Gew.-% bis 100 Gew.%; |
| • n-Butan: | 0 Gew.-% bis 100 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Summe sonstige Stoffe: | 0 Gew.-% bis 5 Gew.%; |
b) Abmischen eines Einsatzgemisches unter Verwendung des LPG bzw. des NGL;
c) für den Fall, dass das Einsatzgemisch mehr als 1.0 Gew.-% ungesättigte Kohlenwasserstoffe enthält: Reduzieren des Gehalts ungesättigter Kohlenwasserstoffe im Einsatzgemisch auf einen Wert unter 1.0 Gew.-% durch Unterwerfen des Einsatzgemisches einer Hydrierung;
d) optional: Reduzieren des Gehalts an n-Butan des Einsatzstoffgemisches durch Destillation des Einsatzgemisches unter Erhalt einer n-Butan enthaltenden Sumpffraktion, wobei der der Anteil an n-Butan in der n-Butan enthaltenden Sumpffraktion größer ist als der Anteil an n-Butan in dem destillierten Einsatzgemisch;
e) Dehydrieren des Einsatzgemisches unter Erhalt von mindestens einem Dehydrierungsgemisch,
f) Gewinnen einer C₄-Fraktion aus dem Dehydrierungsgemisch, wobei die C₄-Fraktion die folgende, sich zu 100 Gew.-% ergänzende, die Grenzwerte einschließende Zusammensetzung aufweist:
| | |
|---|---|
| • 1.3-Butadien: | 1 Gew.-% bis 5 Gew.-%; |
| • Isobuten: | 20 Gew.-% bis 50 Gew.-%; |
| • n-Buten: | 20 Gew.-% bis 50 Gew.-%; |
| • Summe Isobutan und n-Butan: | 2 Gew.-% bis 60 Gew.-%; |
| • Summe sonstige Stoffe: | 0 Gew.-% bis 1 Gew.-%; |
g) zumindest teilweises Entfernen von 1.3-Butadien und Isobuten aus der C₄-Fraktion unter Erhalt eines Intermediats, welches die folgende sich zu 100 Gew.-% ergänzende, die Grenzwerte einschließende Zusammensetzung aufweist:
| | |
|---|---|
| • 1.3-Butadien: | 0 Gew.-% bis 500 Gew.-ppm; |
| • Isobuten: | 0 Gew.-% bis 2 Gew.-%; |
| • n-Buten: | 30 Gew.-% bis 55 Gew.-%; |
| • Summe Isobutan und n-Butan: | 45 Gew.-% bis 70 Gew.-%; |
| • Summe sonstige Stoffe: | 0 Gew.-% bis 500 Gew.-ppm; |
h) Aufteilen des Intermediats in eine erste Portion und in eine zweite Portion in einem Splitter;
i) optional Reduzieren des Gehalts an Isobutan der ersten Portion des Intermediats durch Destillation der ersten Portion des Intermediats unter Erhalt einer Isobutan enthaltenden Sumpffraktion, wobei der der Anteil an Isobutan in der Isobutan enthaltenden Sumpffraktion größer ist als der Anteil an Isobutan in der destillierten ersten Portion des Intermediats und Verwenden der Isobutan enthaltenden Sumpffraktion zum Erhöhen des Gehalts an Isobutan des Einsatzgemisches, indem die Isobutan enthaltenden Sumpffraktion dem Einsatzgemisch zugegeben wird oder beim Abmischen des Einsatzgemisches verwendet wird;
j) Unterwerfen der ersten Portion des Intermediats einer Oligomerisierung in Gegenwart eines festen Katalysators, der amorphes Silicaalumina und mindestens 15 Gew.-% Nickel enthält, wodurch ein Oligomerisat erhalten wird, welches Olefine mit acht Kohlenstoffatomen und Butan enthält,
k) Abtrennen von Butan aus dem Oligomerisat und Verwenden des abgetrennten Butans beim Abmischen des Einsatzgemisches;
l) Abtrennen von Olefinen mit acht Kohlenstoffatomen aus dem Oligomerisat und Beaufschlagen der abgetrennten Olefinen mit acht Kohlenstoffatomen mit Synthesegas zwecks Durchführung einer ersten Hydroformylierung unter Erhalt eines ersten Hydroformylierungsgemisches, welches zumindest Aldehyde mit neun Kohlenstoffatome enthält;
m) Abtrennen einer ersten Zielfraktion enthaltend Aldehyde mit neun Kohlenstoffatomen aus dem ersten Hydroformylierungsgemisch;
n) Beaufschlagen der zweiten Portion des Intermediats mit Synthesegas zwecks Durchführung einer zweiten Hydroformylierung unter Erhalt eines zweiten Hydroformylierungsgemisches, welches zumindest Aldehyde mit fünf Kohlenstoffatome sowie Butan enthält;
o) Abtrennen von Butan aus dem zweiten Hydroformylierungsgemisch und Verwenden des abgetrennten Butans beim Abmischen des Einsatzgemisches;
p) Abtrennen einer zweiten Zielfraktion enthaltend Aldehyde mit fünf Kohlenstoffatomen aus dem zweiten Hydroformylierungsgemisch.

2. Verfahren nach Anspruch 1, **dadurch gekennzeichnet, dass** das Aufteilen des Intermediats in die erste Portion und in die zweite Portion unter Berücksichtigung eines sich zeitlich ändernden Bedarfs an Aldehyd mit neun Kohlenstoffatomen und an Aldehyd mit fünf Kohlenstoffatomen erfolgt, dergestalt, dass das Mengenverhältnis von erster Portion zu zweiter Portion sich mit der Zeit ändert analog zu der zeitlichen Änderung des Verhältnisses des Bedarfs an Aldehyd mit neun Kohlenstoffatomen zu dem Bedarf an Aldehyd mit fünf Kohlenstoffatomen.

3. Verfahren nach Anspruch 1 oder 2, wobei das Oligomerisat auch nicht umgesetzte Olefine mit vier Kohlenstoffatome enthält, **dadurch gekennzeichnet, dass** nicht umgesetzte Olefine mit vier Kohlenstoffatome aus dem Oligomerisat abgetrennt und zusammen mit frischem Intermediat in die Oligomerisierung gefahren wird.

4. Verfahren nach Anspruch 3, wobei die Oligomerisierung einer Reaktionszone durchgeführt wird, **dadurch gekennzeichnet, dass** mehr als 60% bis maximal 90 % der in der Oligomerisierung entstehenden Reaktionswärme mit dem Oligomerisat aus der Reaktionszone abgeführt wird (teiladiabate Reaktionsführung).

5. Verfahren nach Anspruch 3, wobei die Oligomerisierung einer Reaktionszone durchgeführt wird **dadurch gekennzeichnet, dass** mehr als 90% bis maximal 100 % der in der Oligomerisierung entstehenden Reaktionswärme mit dem Oligomerisat aus der Reaktionszone abgeführt wird (adiabate Reaktionsführung).

6. Verfahren nach Anspruch 1 oder einem der Ansprüche 2 bis 5, **dadurch gekennzeichnet, dass** das Intermediat dadurch erhalten wird, dass die C₄-Fraktion einer Selektivhydrierung unterzogen wird, um in der C₄-Fraktion enthaltendes 1.3-Butadien durch Hydrierung zumindest teilweise zu entfernen.

7. Verfahren nach Anspruch 6, **dadurch gekennzeichnet, dass** das Intermediat dadurch erhalten wird, dass vor oder nach Durchführung der Selektivhydrierung das in der C₄-Fraktion enthaltende Isobuten mit einem Alkohol zumindest teilweise in einen Ether umgesetzt wird und der dabei gebildete Ether zumindest teilweise destillativ abgetrennt wird.

8. Verfahren nach Anspruch 1 oder einem der Ansprüche 2 bis 7, **dadurch gekennzeichnet, dass** beim Abmischen des Einsatzgemisches zusätzlich Isobutan und/oder n-Butan verwendet wird, welches weder aus dem bereitgestellten LPG bzw. NGL noch aus dem Oligomerisat oder aus dem zweiten Hydroformylierungsgemisch stammt.

9. Verfahren nach Anspruch 1 oder einem der Ansprüche 2 bis 8, **dadurch gekennzeichnet, dass** die Dehydrierung zumindest abschnittsweise ohne Zugabe eines Oxidationsmittels erfolgt.

10. Verfahren nach Anspruch 1 oder einem der Ansprüche 2 bis 9, wobei die Dehydrierung des Einsatzgemisches in einer Reaktionszone erfolgt, **dadurch gekennzeichnet, dass** die im Einsatzgemisch enthaltenden Alkane mit drei und vier Kohlenstoffatomen gemeinsam in derselben Reaktionszone dehydriert werden.

11. Verfahren nach Anspruch 1 oder einem der Ansprüche 2 bis 9, **dadurch gekennzeichnet, dass** die Dehydrierung des Einsatzgemisches in mindestens zwei Reaktionszonen erfolgt, wobei im Einsatzgemisch enthaltenden Alkane mit drei Kohlenstoffatomen in der ersten Reaktionszone dehydriert werden, und wobei im Einsatzgemisch enthaltenden Alkane mit vier Kohlenstoffatomen in der zweiten Reaktionszone dehydriert werden.

12. Verfahren nach Anspruch 10 oder 11, bei welchem der Gehalt an n-Butan des Einsatzgemisches durch Destillation des Einsatzgemisches unter Erhalt einer n-Butan enthaltenden Sumpffraktion dergestalt reduziert wird, dass der der Anteil an n-Butan in der n-Butan enthaltenden Sumpffraktion größer ist als der Anteil an n-Butan in dem destillierten Einsatzgemisch, **dadurch gekennzeichnet, dass** eine weitere Reaktionszone vorgesehen ist, in welcher die Dehydrierung des destillierten Einsatzgemisches nicht erfolgt, wohingegen die n-Buten enthaltende Sumpffraktion in der weiteren Reaktionszone einer Dehydrierung unterworfen wird.

13. Verfahren nach Anspruch 1 oder einem der Ansprüche 2 bis 12, **dadurch gekennzeichnet**, das das Gewinnen der C₄-Fraktion aus dem Dehydrierungsgemisch bzw. den Dehydrierungsgemischen dadurch erfolgt, dass das Dehydrierungsgemisch bzw. die Dehydrierungsgemische gemeinsam destilliert werden, wobei die C₄-Fraktion als Schwersieder zurück bleiben und mindestens eine Leichtsiederfraktion gewonnen wird.

14. Verfahren nach Anspruch 1 oder einem der Ansprüche 2 bis 13, **dadurch gekennzeichnet, dass** das bereitgestellte LPG (Liquefied Petroleum Gas) die folgende, sich zu 100 Gew.-% ergänzende, die Grenzwerte einschließende Zusammensetzung aufweist:
| | |
|---|---|
| • Propan: | 0 Gew.-% bis 3 Gew.%; |
| • Isobutan: | 20 Gew.-% bis 80 Gew.%; |
| • n-Butan: | 20 Gew.-% bis 80 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Summe sonstige Stoffe: | 0 Gew.-% bis 5 Gew.%. |

15. Verfahren nach Anspruch 1 oder einem der Ansprüche 2 bis 13, **dadurch gekennzeichnet, dass** das bereitgestellte LPG (Liquefied Petroleum Gas) die folgende, sich zu 100 Gew.-% ergänzende, die Grenzwerte einschließende Zusammensetzung aufweist:
| | |
|---|---|
| • Propan: | 10 Gew.-% bis 40 Gew.%; |
| • Isobutan: | 15 Gew.-% bis 85 Gew.%; |
| • n-Butan: | 15 Gew.-% bis 85 Gew.%; |
| • Propen: | 0 Gew.-% bis 3 Gew.%; |
| • Isobuten: | 3 Gew.-% bis 10 Gew.%; |
| • n-Buten: | 2 Gew.-% bis 15 Gew.%; |
| • Summe sonstige Stoffe: | 0 Gew.-% bis 5 Gew.%. |

16. Verfahren nach Anspruch 1 oder einem der Ansprüche 2 bis 13, **dadurch gekennzeichnet, dass** das bereitgestellte NGL (Natural Gas Liquids) die folgende, sich zu 100 Gew.-% ergänzende, die Grenzwerte einschließende Zusammensetzung aufweist:
| | |
|---|---|
| • Ethan: | 0 Gew.-% bis 2 Gew.%; |
| • Propan: | 0 Gew.-% bis 50 Gew.%; |
| • Isobutan: | 0 Gew.-% bis 100 Gew.%; |
| • n-Butan: | 0 Gew.-% bis 100 Gew.%; |
| • Propen: | 0 Gew.-% bis 3 Gew.%; |
| • Isobuten: | 0 Gew.-% bis 10 Gew.%; |
| • n-Buten: | 0 Gew.-% bis 15 Gew.%; |
| • Pentan: | 0 Gew.-% bis 2 Gew.%; |
| • Summe sonstige Stoffe: | 0 Gew.-% bis 1 Gew.%. |

## Claims

1. Process for the flexible preparation of aldehydes having five and nine carbon atoms comprising the following steps:
a) providing a liquid mixture, called LPG or NGL (liquefied petroleum gas or natural gas liquids), which comprises specifically a main component selected from the group consisting of propane, isobutane and n-butane, and at least one secondary component selected from the group consisting of propane, isobutane, n-butane, propene, isobutene and n-butene, with the proviso that the main component selected and the secondary component selected are not identical, and that the mixture has the following composition including the limit values which add up to 100% by weight:
| | |
|---|---|
| • propane: | 0 wt% to 50 wt%; |
| • isobutane: | 0 wt% to 100 wt%; |
| • n-butane: | 0 wt% to 100 wt%; |
| • propene: | 0 wt% to 3 wt%; |
| • isobutene: | 0 wt% to 10 wt%; |
| • n-butene: | 0 wt% to 15 wt%; |
| • sum of other substances: | 0 wt% to 5 wt%; |
b) mixing a feed mixture using the LPG or NGL;
c) in the case that the feed mixture comprises more than 1.0% by weight unsaturated hydrocarbons: reducing the content of unsaturated hydrocarbons in the feed mixture to a value below 1.0% by weight by subjecting the feed mixture to a hydrogenation;
d) optionally: reducing the n-butane content of the feedstock mixture by distillation of the feed mixture to obtain a bottoms fraction comprising n-butane, wherein the proportion of n-butane in the bottoms fraction comprising n-butane is greater than the proportion of n-butane in the distilled feed mixture;
e) dehydrogenating the feed mixture to obtain at least one dehydrogenation mixture,
f) obtaining a C₄ fraction from the dehydrogenation mixture, wherein the C₄ fraction has the following composition including the limit values which add up to 100% by weight:
| | |
|---|---|
| • 1,3-butadiene: | 1 wt% to 5 wt%; |
| • isobutene: | 20 wt% to 50 wt%; |
| • n-butene: | 20 wt% to 50 wt%; |
| • sum of isobutane andn-butane: | 2 wt% to 60 wt% ; |
| • sum of other substances: | 0 wt% to 1 wt%; |
g) at least partial removal of 1,3-butadiene and isobutene from the C₄ fraction to obtain an intermediate which has the following composition including the limit values which add up to 100% by weight:
| | |
|---|---|
| • 1,3-butadiene: | 0 wt% to 500 ppm by weight; |
| • isobutene: | 0 wt% to 2 wt%; |
| • n-butene: | 30 wt% to 55 wt%; |
| • sum of isobutane andn-butane: | 45 wt% to 70 wt%; |
| • sum of other substances: | 0 wt% to 500 ppm byweight; |
h) dividing the intermediate into a first portion and a second portion in a splitter;
i) optionally reducing the isobutane content of the first portion of the intermediate by distillation of the first portion of the intermediate to obtain a bottoms fraction comprising isobutane, wherein the proportion of isobutane in the bottoms fraction comprising isobutane is greater than the proportion of isobutane in the first portion of the intermediate distilled and using the bottoms fraction comprising isobutane to increase the isobutane content of the feed mixture in which the bottoms fraction comprising isobutane is added to the feed mixture or is used when mixing the feed mixture;
j) subjecting the first portion of the intermediate to an oligomerization in the presence of a solid catalyst comprising amorphous silica/alumina and at least 15% by weight nickel, whereby an oligomer is obtained comprising olefins having eight carbon atoms and butane,
k) separating butane from the oligomer and using the butane separated when mixing the feed mixture;
l) separating olefins having eight carbon atoms from the oligomer and pressurizing the separated olefins having eight carbon atoms with synthesis gas for the purpose of carrying out a first hydroformylation to obtain a first hydroformylation mixture comprising at least aldehydes having nine carbon atoms;
m) separating a first target fraction comprising aldehydes having nine carbon atoms from the first hydroformylation mixture;
n) pressurizing the second portion of the intermediate with synthesis gas for the purpose of carrying out a second hydroformylation to obtain a second hydroformylation mixture comprising at least aldehydes having five carbon atoms and also butane;
o) separating butane from the second hydroformylation mixture and using the butane separated when mixing the feed mixture;
p) separating a second target fraction comprising aldehydes having five carbon atoms from the second hydroformylation mixture.

2. Process according to Claim 1, **characterized in that** the intermediate is divided into the first portion and the second portion considering the changing need over time for aldehyde having nine carbon atoms and aldehyde having five carbon atoms in such a way that the ratio of the first portion to the second portion changes analogously with time to the change over time of the ratio of the need for aldehyde having nine carbon atoms to the need for aldehyde having five carbon atoms.

3. Process according to Claim 1 or 2, wherein the oligomer also comprises unreacted olefins having four carbon atoms, **characterized in that** unreacted olefins having four carbon atoms are separated from the oligomer and are fed into the oligomerization together with fresh intermediate.

4. Process according to Claim 3, wherein the oligomerization is conducted in a reaction zone, **characterized in that** more than 60% up to at most 90% of the heat of reaction resulting from the oligomerization is removed from the reaction zone with the oligomer (partially adiabatic reaction regime).

5. Process according to Claim 3, wherein the oligomerization is conducted in a reaction zone, **characterized in that** more than 90% up to at most 100% of the heat of reaction resulting from the oligomerization is removed from the reaction zone with the oligomer (adiabatic reaction regime).

6. Process according to Claim 1 or any of Claims 2 to 5, **characterized in that** the intermediate is obtained by subjecting the C₄ fraction to a selective hydrogenation in order to at least partially remove 1,3-butadiene present in the C₄ fraction by hydrogenation.

7. Process according to Claim 6, **characterized in that** the intermediate is obtained, before or after carrying out the selective hydrogenation, by reacting the isobutene present in the C₄ fraction with an alcohol at least partially to give an ether and removing the ether formed in this case at least partially by distillation.

8. Process according to Claim 1 or any of Claims 2 to 7, **characterized in that** additionally isobutane and/or n-butane is used when mixing the feed mixture, which does not originate from the LPG or NGL provided nor from the oligomer or from the second hydroformylation mixture.

9. Process according to Claim 1 or any of Claims 2 to 8, **characterized in that** the dehydrogenation is effected at least partially without addition of an oxidizing agent.

10. Process according to Claim 1 or any of Claims 2 to 9, wherein the dehydrogenation of the feed mixture is conducted in a reaction zone, **characterized in that** the alkanes having three and four carbon atoms present in the feed mixture are dehydrogenated together in the same reaction zone.

11. Process according to Claim 1 or any of Claims 2 to 9, **characterized in that** the dehydrogenation of the feed mixture is conducted in at least two reaction zones, wherein alkanes having three carbon atoms present in the feed mixture are dehydrogenated in the first reaction zone and wherein alkanes having four carbon atoms present in the feed mixture are dehydrogenated in the second reaction zone

12. Process according to Claim 10 or 11, in which the n-butane content of the feed mixture is reduced by distillation of the feed mixture to obtain a bottoms fraction comprising n-butane such that the proportion of n-butane in the bottoms fraction comprising n-butane is greater than the proportion of n-butane in the distilled feed mixture, **characterized in that** a further reaction zone is provided in which the the distilled feed mixture is not dehydrogenated whereas the bottoms fraction comprising n-butene is subjected to a dehydrogenation in the further reaction zone.

13. Process according to Claim 1 or any of Claims 2 to 12, **characterized in that** the C₄ fraction is obtained from the dehydrogenation mixture or dehydrogenation mixtures by distilling the dehydrogenation mixture or dehydrogenation mixtures together, wherein the C₄ fraction remains as high boilers and at least one low-boiler fraction is obtained.

14. Process according to Claim 1 or any of Claims 2 to 13, **characterized in that** the LPG (liquefied petroleum gas) provided has the following composition including the limit values adding up to 100% by weight:
| | |
|---|---|
| • propane: | 0 wt% to 3 wt%; |
| • isobutane: | 20 wt% to 80 wt%; |
| • n-butane: | 20 wt% to 80 wt%; |
| • propene: | 0 wt% to 3 wt%; |
| • isobutene: | 0 wt% to 10 wt%; |
| • n-butene: | 0 wt% to 15 wt%; |
| • sum of other substances: | 0 wt% to 5 wt%. |

15. Process according to Claim 1 or any of Claims 2 to 13, **characterized in that** the LPG (liquefied petroleum gas) provided has the following composition including the limit values adding up to 100% by weight:
| | |
|---|---|
| • propane: | 10 wt% to 40 wt%; |
| • isobutane: | 15 wt% to 85 wt%; |
| • n-butane: | 15 wt% to 85 wt%; |
| • propene: | 0 wt% to 3 wt%; |
| • isobutene: | 3 wt% to 10 wt%; |
| • n-butene: | 2 wt% to 15 wt%; |
| • sum of other substances: | 0 wt% to 5 wt%. |

16. Process according to Claim 1 or any of Claims 2 to 13, **characterized in that** the NGL (natural gas liquids) provided has the following composition including the limit values adding up to 100% by weight:
| | |
|---|---|
| • ethane: | 0 wt% to 2 wt%; |
| • propane: | 0 wt% to 50 wt%; |
| • isobutane: | 0 wt% to 100 wt%; |
| • n-butane: | 0 wt% to 100 wt%; |
| • propene: | 0 wt% to 3 wt%; |
| • isobutene: | 0 wt% to 10 wt%; |
| • n-butene: | 0 wt% to 15 wt%; |
| • pentane: | 0 wt% to 2 wt%; |
| • sum of other substances: | 0 wt% to 1 wt%. |

## Revendications

1. Procédé pour la fabrication souple d'aldéhydes comprenant cinq et neuf atomes de carbone, qui présente les étapes suivantes :
a) mise à disposition d'un mélange liquide, appelé LPG ou NGL (Liquefied Petroleum Gas/gaz de pétrole liquéfié ou Natural Gas Liquids/liquide de gaz naturel), qui comprend exactement un composant principal, choisi dans le groupe constitué par le propane, l'isobutane et le n-butane, ainsi qu'au moins un composant secondaire, choisi dans le groupe constitué par le propane, l'isobutane, le n-butane, le propène, l'isobutène et le n-butène, étant entendu que le composant principal choisi et le composant secondaire choisi ne sont pas identiques et que le mélange présente la composition suivante, se complétant à 100% en poids, les valeurs limites étant incluses :
| | |
|---|---|
| - propane : | 0% en poids à 50% en poids ; |
| - isobutane : | 0% en poids à 100% en poids ; |
| - n-butane : | 0% en poids à 100% en poids ; |
| - propène : | 0% en poids à 3% en poids ; |
| - isobutène : | 0% en poids à 10% en poids ; |
| - n-butène : | 0% en poids à 15% en poids ; |
| - somme des autres substances : | 0% en poids à 5% enpoids; |
b) mélange d'un mélange de départ avec utilisation du LPG ou du NGL ;
c) pour le cas où le mélange de départ contient plus de 1,0% en poids d'hydrocarbures insaturés : réduction de la teneur en hydrocarbures insaturés dans le mélange de départ à une valeur inférieure à 1,0% en poids par soumission du mélange de départ à une hydrogénation ;
d) facultativement : réduction de la teneur en n-butane du mélange de substances de départ par distillation du mélange de départ avec obtention d'une fraction de fond contenant du n-butane, la proportion de n-butane dans la fraction de fond contenant du n-butane étant supérieure à la proportion de n-butane dans le mélange de départ distillé ;
e) déshydrogénation du mélange de départ avec obtention d'au moins un mélange déshydrogéné,
f) obtention d'une fraction en C₄ à partir du mélange de déshydrogénation, la fraction en C₄ présentant la composition suivante, se complétant à 100% en poids, les valeurs limites étant incluses :
| | |
|---|---|
| - 1,3-butadiène : | 1% en poids à 5% en poids ; |
| - isobutène : | 20% en poids à 50% en poids ; |
| - n-butène : | 20% en poids à 50% en poids ; |
| - somme d'isobutane à 60% et de n-butane : | 2% en poids enpoids; |
| - somme des autres substances : | 0% en poids à 1% enpoids; |
g) élimination au moins partielle du 1,3-butadiène et de l'isobutène à partir de la fraction en C₄ avec obtention d'un intermédiaire qui présente la composition suivante, se complétant à 100% en poids, les valeurs limites étant incluses :
| | |
|---|---|
| - 1,3-butadiène : | 0% en poids à 500 ppm en poids; |
| - isobutène : | 0% en poids à 2% en poids ; |
| - n-butène : | 30% en poids à 55% en poids ; |
| - somme d'isobutane et de n-butane: | 45% en poids à 70% en poids ; |
| - somme des autres substances : | 0% en poids à 500 ppm en poids; |
h) division de l'intermédiaire en une première portion et en une deuxième portion dans une tour de fractionnement ;
i) facultativement : réduction de la teneur en isobutane la première portion de l'intermédiaire par distillation de la première portion de l'intermédiaire avec obtention d'une fraction de fond contenant de l'isobutane, la proportion d'isobutane dans la fraction de fond contenant de l'isobutane étant supérieure à la proportion d'isobutane dans la première portion distillée de l'intermédiaire et utilisation de la fraction de fond contenant de l'isobutane pour augmenter la teneur en isobutane du mélange de départ en ce que la fraction de fond contenant de l'isobutane est introduite dans le mélange de départ ou est utilisée lors du mélange du mélange de départ ;
j) soumission de la première portion de l'intermédiaire à une oligomérisation en présence d'un catalyseur solide, qui contient de la silice-alumine amorphe et au moins 15% de nickel, ce qui permet d'obtenir un oligomère qui contient des oléfines comprenant huit atomes de carbone et du butane,
k) séparation du butane de l'oligomère et utilisation du butane séparé lors du mélange du mélange de départ ;
l) séparation des oléfines comprenant huit atomes de carbone de l'oligomère et soumission des oléfines séparées comprenant huit atomes de carbone à l'action d'un gaz de synthèse en vue de réaliser une première hydroformylation avec obtention d'un premier mélange d'hydroformylation, qui contient au moins des aldéhydes comprenant neuf atomes de carbone ;
m) séparation d'une première fraction cible contenant des aldéhydes comprenant neuf atomes de carbone du premier mélange d'hydroformylation ;
n) soumission de la deuxième portion de l'intermédiaire à l'action d'un gaz de synthèse en vue de réaliser une deuxième hydroformylation avec obtention d'un deuxième mélange d'hydroformylation, qui contient au moins des aldéhydes comprenant cinq atomes de carbone ainsi que du butane ;
o) séparation du butane du deuxième mélange d'hydroformylation et utilisation du butane séparé lors du mélange du mélange de départ ;
p) séparation d'une deuxième fraction cible contenant des aldéhydes comprenant cinq atomes de carbone du deuxième mélange d'hydroformylation.

2. Procédé selon la revendication 1, **caractérisé en ce que** la division de l'intermédiaire en la première portion et la deuxième portion tout en tenant compte du besoin qui se modifie dans le temps en aldéhyde comprenant neuf atomes de carbone et en aldéhyde comprenant cinq atomes de carbone a lieu de manière telle que le rapport des quantités de la première portion à la deuxième portion se modifie au fil du temps de manière analogue à la modification dans le temps du rapport du besoin en aldéhyde comprenant neuf atomes de carbone au besoin en aldéhyde comprenant cinq atomes de carbone.

3. Procédé selon la revendication 1 ou 2, l'oligomère contenant également des oléfines non transformées comprenant quatre atomes de carbone, **caractérisé en ce que** les oléfines non transformées comprenant quatre atomes de carbone sont séparées de l'oligomère et introduites, conjointement avec de l'intermédiaire frais, dans l'oligomérisation.

4. Procédé selon la revendication 3, l'oligomérisation étant réalisée dans une zone de réaction, **caractérisé en ce que** plus de 60% jusqu'à maximum 90% de la chaleur de réaction formée lors de l'oligomérisation sont évacués avec l'oligomère hors de la zone de réaction (conduite partiellement adiabatique de la réaction).

5. Procédé selon la revendication 3, l'oligomérisation étant réalisée dans une zone de réaction, **caractérisé en ce que** plus de 90% jusqu'à maximum 100% de la chaleur de réaction formée lors de l'oligomérisation sont évacués avec l'oligomère hors de la zone de réaction (conduite adiabatique de la réaction).

6. Procédé selon la revendication 1 ou selon l'une quelconque des revendications 2 à 5, **caractérisé en ce que** l'intermédiaire est obtenu **en ce que** la fraction en C₄ est soumise à une hydrogénation sélective afin d'éliminer au moins partiellement le 1,3-butadiène contenu dans la fraction en C₄ par hydrogénation.

7. Procédé selon la revendication 6, **caractérisé en ce que** l'intermédiaire est obtenu **en ce que**, avant ou après la réalisation de l'hydrogénation sélective, l'isobutène contenu dans la fraction en C₄ est transformé au moins partiellement avec un alcool en un éther et l'éther ainsi formé est séparé au moins partiellement par distillation.

8. Procédé selon la revendication 1 ou selon l'une quelconque des revendications 2 à 7, **caractérisé en ce que** lors du mélange du mélange de départ, de l'isobutane et/ou du n-butane est/sont utilisé (s) en plus, qui ne provien(nen)t ni du LPG ou du NGL mis à disposition, ni de l'oligomère ou du deuxième mélange d'hydroformylation.

9. Procédé selon la revendication 1 ou selon l'une quelconque des revendications 2 à 8, **caractérisé en ce que** la déshydrogénation est effectuée au moins en partie sans addition d'un oxydant.

10. Procédé selon la revendication 1 ou selon l'une quelconque des revendications 2 à 9, la déshydrogénation du mélange de départ ayant lieu dans une zone de réaction, **caractérisé en ce que** les alcanes comprenant trois et quatre atomes de carbone contenus dans le mélange de départ sont déshydrogénés conjointement dans la même zone de réaction.

11. Procédé selon la revendication 1 ou selon l'une quelconque des revendications 2 à 9, **caractérisé en ce que** la déshydrogénation du mélange de départ a lieu dans au moins deux zones de réaction, les alcanes contenant trois atomes de carbone contenus dans le mélange de départ étant déshydrogénés dans la première zone de réaction et les alcanes comprenant quatre atomes de carbone contenus dans la mélange de départ étant déshydrogénés dans la deuxième zone de réaction.

12. Procédé selon la revendication 10 ou 11, dans lequel la teneur en n-butane du mélange de départ est réduite par distillation du mélange de départ avec obtention d'une fraction de fond contenant du n-butane de manière telle que la proportion de n-butane dans la fraction de fond contenant du n-butane est supérieure à la proportion de n-butane dans le mélange de départ distillé, **caractérisé en ce qu'**une autre zone de réaction est prévue, dans laquelle la déshydrogénation du mélange de départ distillé ne se produit pas, alors que la fraction de fond contenant du n-butane est soumise à une déshydrogénation dans l'autre zone de réaction.

13. Procédé selon la revendication 1 ou selon l'une quelconque des revendications 2 à 12, **caractérisé en ce que** l'obtention de la fraction en C₄ à partir du mélange de déshydrogénation ou des mélanges de déshydrogénation a lieu **en ce que** le mélange de déshydrogénation ou les mélanges de déshydrogénation sont distillés en commun, la fraction en C₄ restant comme fraction à point d'ébullition élevé et au moins une fraction à bas point d'ébullition étant obtenue.

14. Procédé selon la revendication 1 ou selon l'une quelconque des revendications 2 à 13, **caractérisé en ce que** le LPG (Liquefied Petroleum Gas) mis à disposition présente la composition suivante, se complétant à 100% en poids, les valeurs limites étant incluses :
| | |
|---|---|
| - propane : | 0% en poids à 3% en poids ; |
| - isobutane : | 20% en poids à 80% en poids ; |
| - n-butane : | 20% en poids à 80% en poids ; |
| - propène : | 0% en poids à 3% en poids ; |
| - isobutène : | 0% en poids à 10% en poids ; |
| - n-butène : | 0% en poids à 15% en poids ; |
| - somme des autres substances : | 0% en poids à 5% en poids. |

15. Procédé selon la revendication 1 ou selon l'une quelconque des revendications 2 à 13, **caractérisé en ce que** le LPG (Liquefied Petroleum Gas) mis à disposition présente la composition suivante, se complétant à 100% en poids, les valeurs limites étant incluses :
| | |
|---|---|
| - propane : | 10% en poids à 40% en poids ; |
| - isobutane : | 15% en poids à 85% en poids ; |
| - n-butane : | 15% en poids à 85% en poids ; |
| - propène : | 0% en poids à 3% en poids ; |
| - isobutène : | 3% en poids à 10% en poids ; |
| - n-butène : | 2% en poids à 15% en poids ; |
| - somme des autres substances : | 0% en poids à 5% en poids. |

16. Procédé selon la revendication 1 ou selon l'une quelconque des revendications 2 à 13, **caractérisé en ce que** le NGL (Natural Gas Liquids) mis à disposition présente la composition suivante, se complétant à 100% en poids, les valeurs limites étant incluses :
| | |
|---|---|
| - éthane : | 0% en poids à 2% en poids ; |
| - propane : | 0% en poids à 50% en poids ; |
| - isobutane : | 0% en poids à 100% en poids ; |
| - n-butane : | 0% en poids à 100% en poids ; |
| - propène : | 0% en poids à 3% en poids ; |
| - isobutène : | 0% en poids à 10% en poids ; |
| - n-butène : | 0% en poids à 15% en poids ; |
| - pentane : | 0% en poids à 2% en poids ; |
| - somme des autres substances : | 0% en poids à 1% en poids. |
